(19) Europäisches Patentamt
European Patent Office
Office européen des brevets

(11) Veröffentlichungsnummer: **0 025 941**
**B1**

# (12) EUROPÄISCHE PATENTSCHRIFT

(45) Veröffentlichungstag der Patentschrift: **04.05.83**

(51) Int. Cl.[3]: **C 07 D 487/04,** C 07 D 237/04, A 61 K 31/50 // (C07D487/04, 237/00)

(21) Anmeldenummer: **80105411.5**

(22) Anmeldetag: **10.09.80**

(54) **Pyridazopyridazinderivate, Verfahren zu deren Herstellung und Arzneimittel enthaltend solche Pyridazopyridazinderivate.**

(30) Priorität: **19.09.79 GB 7932531**
**11.07.80 GB 8022701**

(43) Veröffentlichungstag der Anmeldung: **01.04.81 Patentblatt 81/13**

(45) Bekanntmachung des Hinweises auf die Patenterteilung: **04.05.83 Patentblatt 83/18**

(84) Benannte Vertragsstaaten: **AT BE CH DE FR GB IT LI LU NL SE**

(56) Entgegenhaltungen:
**AT-B-322 562**
**CH-A-593 284**

(73) Patentinhaber: **F. HOFFMANN-LA ROCHE & CO. Aktiengesellschaft, CH-4002 Basel (CH)**

(72) Erfinder: **Hassal,Cedric Herbert, 23 The Ryde, Hatfield, Herts. AL9 5 DL (GB)**
Erfinder: **Moody, Christopher John, 3 Damask Close Weston, Stevenage, Herts (GB)**

(74) Vertreter: **Lederer, Franz, Dr. et al, Patentanwälte Dr. Franz Lederer Reiner F. Meyer Lucile-Grahn-Strasse 22, D-8000 München 80 (DE)**

EP 0 025 941 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Pyridazopyridazinderivate, Verfahren zu deren Herstellung und Arzneimittel enthaltend solche Pyridazopyridazinderivate

In der schweizerischen Patentschrift Nr. 593 284 ist das anti-inflammatorisch wirksame Phthalazino(2,3-b)phthalazin-5(14H),12(7H)-dion sowie dessen Herstellung unter Verwendung von 7-Hydroxy- und 7-Alkoxy-phthalazino(2,3-b)phthalazin-5(14H),12(7H)-dionen als Zwischenprodukte beschrieben.

Aus der österreichischen Patentschrift Nr. 322 562 sind Pyrido(3,4-d)pyridazinderivate bekannt, welche als Diuretika verwendet werden können. Demgegenüber betrifft die vorliegende Erfindung Pyridazopyridazinderivate mit wertvollen antihypertensiven Eigenschaften.

Die erfindungsgemäßen Pyridazopyridazin-Derivate sind Verbindungen der allgemeinen Formel

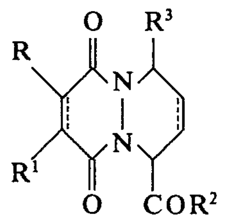

(I)

worin einer der Reste R und $R^1$ Wasserstoff oder $C_1-C_6$-Alkyl und der andere einen Rest der Formel

$$-(A)_n-Y \qquad (i)$$

bedeutet, wobei A eine gegebenenfalls durch $C_1-C_6$-Alkyl substituierte Methylen,- Äthylen- oder Propylengruppe, Y Mercapto, $C_2-C_6$-Alkanoylthio, Benzoylthio oder Benzylthio und n die Zahl 0 oder 1 bedeuten, $R^2$ Hydroxy, $C_1-C_6$-Alkoxy oder Amino, $R^3$ Wasserstoff, $C_1-C_6$-Alkyl oder gegebenenfalls durch $C_1-C_6$-Alkyl, $C_1-C_6$-Alkoxy, Halogen oder Trifluormethyl substituiertes Phenyl und die gestrichelten Linien fakultative Bindungen bedeuten,
und Salze von Carbonsäuren der allgemeinen Formel I mit pharmazeutisch annehmbaren Basen.

Der in der vorliegenden Beschreibung verwendete Ausdruck »$C_1-C_6$-Alkyl«, für sich allein genommen oder in Kombinationen, bedeutet geradkettige oder verzweigte Alkylreste, wie Methyl, Äthyl, Propyl, Isopropyl, Butyl, t-Butyl, Pentyl und Hexyl. Der Ausdruck »$C_1-C_6$-Alkoxy« bedeutet geradkettige oder verzweigte Alkoxygruppen, wie Methoxy, Äthoxy, Propoxy, Isopropoxy und dergleichen. Der $C_2-C_6$-Alkanoylrest einer $C_2-C_6$-Alkanoylthio-Gruppe leitet sich von einer geradkettigen oder verzweigten Fettsäure ab, wie Essigsäure, Propionsäure, Buttersäure, Isobuttersäure, Valeriansäure, Pivalinsäure und dergleichen; beispielhaft für $C_2-C_6$-Alkanoylthio-Gruppen sind demnach Acethylthio, Propionylthio und dergleichen. Der Ausdruck »Halogen« bedeutet Fluor, Chlor, Brom oder Jod.

In einer bevorzugten Ausführungsform umfaßt die vorliegende Erfindung Verbindungen der allgemeinen Formel I, worin R Wasserstoff, $R^1$ einen Rest der Formel (i), $R^2$ Hydroxy oder $C_1-C_6$-Alkoxy und $R^3$ Wasserstoff bedeuten und die in 7,8-Stellung eine Einfachbindung besitzen. Der Rest der Formel (i) bedeutet vorzugsweise Mercaptomethyl oder $C_2-C_6$-Alkanoylthiomethyl, wie Acethylthiomethyl.

Besonders bevorzugte Verbindungen der allgemeinen Formel I sind:

(rac.)-Methyl-8-(acetylthio)methyl-octahydro-6,9-dioxo-pyridazo[1,2-a]pyridazin-1-carboxylat und
(rac.)-8-(Mercaptomethyl)-octahydro-6,9-dioxopyridazo[1,2-a]pyridazin-1-carbonsäure.

Weitere bevorzugte Verbindungen der allgemeinen Formel I sind:

(rac.)-t-Butyl-8-(acetylthio)methyl-octahydro-6,9-dioxopyridazo[1,2-a]pyridazin-1-carboxylat,
(rac.)-8-(Acetylthio)methyl-octahydro-6,9-dioxopyridazo[1,2-a]pyridazin-1-carbonsäure,
(rac.)-Methyl-8-(acetylthio)methyl-1,4,6,7,8,9-hexahydro-6,9-dioxopyridazo[1,2-a]pyridazin-1-carboxylat,
(rac.)-8-(Mercaptomethyl)-1,4,6,7,8,9-hexahydro-6,9-dioxopyridazo[1,2-a]pyridazin-1-carbonsäure,
(+)-Methyl-8-(acetylthio)methyl-1,4,6,7,8,9-hexahydro-6,9-dioxopyridazo[1,2-a]pyridazin-1-carboxylat,
(−)-Methyl-8-(acetylthio)methyl-1,4,6,7,8,9-hexahydro-6,9-dioxopyridazo[1,2-a]pyridazin-1-carboxylat und

(−)-8-(Mercaptomethyl)-1,4,6,7,8,9-hexahydro-6,9-dioxopyridazo[1,2-a]pyridazin-1-carbonsäure.

Die Pyridazopyridazin-Derivate der allgemeinen Formel I und ihre Salze mit pharmazeutisch annehmbaren Basen können erfindungsgemäß dadurch hergestellt werden, daß man

a) zur Herstellung einer Verbindung der allgemeinen Formel I, worin $R^2$ $C_1-C_6$-Alkoxy und $R^3$ Wasserstoff bedeuten und die in der 2,3- und der 7,8-Stellung Einfachbindungen besitzt, eine Verbindung der allgemeinen Formel

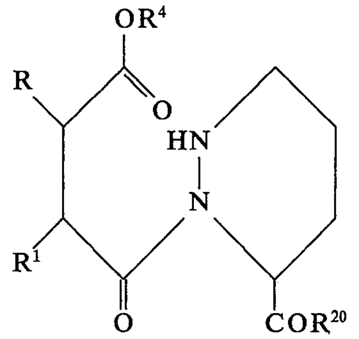
(II)

worin R und $R^1$ obige Bedeutung besitzen, $R^4$ Wasserstoff oder $C_1-C_6$-Alkyl und $R^{20}$ $C_1-C_6$-Alkoxy bedeuten,
cyclisiert, oder

b) zur Herstellung einer Verbindung der allgemeinen Formel I, worin $R^2$ Hydroxy und $R^3$ Wasserstoff bedeuten und die in der 2,3-Stellung eine Einfachbindung besitzt, die Verbindung der Formel

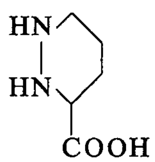
(III)

mit einem Anhydrid der allgemeinen Formel

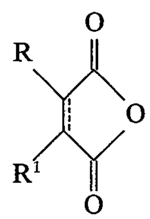
(IV)

worin R, $R^1$ und die gestrichelte Linie obige Bedeutung besitzen,
umsetzt, oder

c) zur Herstellung einer Verbindung der allgemeinen Formel I, die in der 2,3-Stellung eine Doppelbindung besitzt, eine Verbindung der allgemeinen Formel

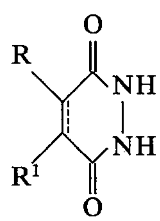
(V).

worin R, $R^1$ und die gestrichelte Linie obige Bedeutung besitzen,
unter oxydierenden Bedingungen mit einer Verbindung der allgemeinen Formel

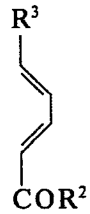

(VIa)

worin $R^2$ und $R^3$ obige Bedeutung besitzen, umsetzt, oder

d) zur Herstellung einer Verbindung der allgemeinen Formel I, worin $R^2$ Hydroxy bedeutet und die in der 2,3-Stellung eine Doppelbindung besitzt, eine Verbindung der allgemeinen Formel

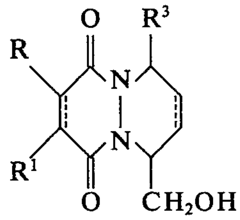

(XVIII)

worin R, $R^1$, $R^3$ und die gestrichelten Linien obige Bedeutung besitzen, oxydiert, oder

e) zur Herstellung einer Verbindung der allgemeinen Formel I, worin Y im Rest der Formel (i) $C_2-C_6$-Alkanoylthio, Benzoylthio oder Benzylthio bedeutet, eine Verbindung der allgemeinen Formel

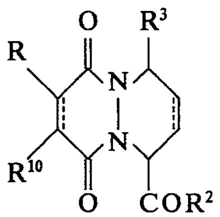

(VII)

worin $R^2$, $R^3$ und die gestrichelten Linien obige Bedeutung besitzen und einer der Reste R und $R^{10}$ Wasserstoff oder $C_1-C_6$-Alkyl und der andere einen Rest der Formel

$$-(A)_n-X \qquad \text{(ii)}$$

bedeutet, wobei A und n obige Bedeutung besitzen und X Halogen bedeutet, mit einer Verbindung der allgemeinen Formel

$$R^6-SH \qquad \text{(VIII)}$$

worin $R^6$ $C_2-C_6$-Alkanoyl, Benzoyl oder Benzyl bedeutet, umsetzt, oder

f) zur Herstellung einer Verbindung der allgemeinen Formel I, worin Y Mercapto bedeutet, in einer Verbindung der allgemeinen Formel I, worin Y $C_2-C_6$-Alkanoylthio, Benzoylthio oder Benzylthio bedeutet, die $C_2-C_6$-Alkanoyl-, Benzoyl- oder Benzyl-Gruppe abspaltet, oder

g) zur Herstellung einer Verbindung der allgemeinen Formel I, worin R Wasserstoff und $R^1$ einen Rest der Formel (i) bedeutet, wobei A eine Methylengruppe, Y $C_2-C_6$-Alkanoylthio oder Benzoylthio und n die Zahl 1 bedeuten, $R_2$ $C_1-C_6$-Alkoxy und $R^3$ Wasserstoff bedeuten, und die in der 2,3- und der 7,8-Stellung Einfachbindungen besitzt, eine Verbindung der allgemeinen Formel

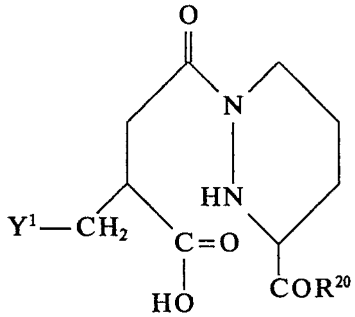

(IX)

worin $R^{20}$ obige Bedeutung besitzt und $Y^1$ $C_2-C_6$-Alkanoylthio oder Benzoylthio bedeutet, cyclisiert, oder

h) zur Herstellung einer Verbindung der allgemeinen Formel I, worin R Wasserstoff und $R^1$ einen Rest der Formel (i) bedeuten, wobei A eine Methylengruppe, Y $C_2-C_6$-Alkanoylthio oder Benzoylthio und n die Zahl 1 bedeuten, und $R^2$ Hydroxy oder $C_1-C_6$-Alkoxy bedeutet und die in der 7,8-Stellung eine Einfachbindung besitzt, eine Verbindung der allgemeinen Formel

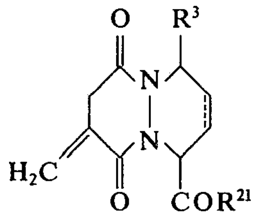

(X)

worin $R^3$ und die gestrichelte Linie obige Bedeutung besitzen und $R^{21}$ Hydroxy oder $C_1-C_6$-Alkoxy bedeutet,
mit einer Verbindung der Formel VIII, worin $R^6$ $C_2-C_6$-Alkanoyl oder Benzoyl bedeutet, umsetzt, oder

i) zur Herstellung einer Verbindung der allgemeinen Formel I, worin $R^2$ $C_1-C_6$-Alkoxy oder Amino bedeutet, eine entsprechende Verbindung der allgemeinen Formel I, worin $R^2$ Hydroxy bedeutet, verestert oder amidiert, oder

j) zur Herstellung einer Verbindung der allgemeinen Formel I, worin $R^2$ Hydroxy bedeutet, eine entsprechende Verbindung der allgemeinen Formel I, worin $R^2$ $C_1-C_6$-Alkoxy bedeutet, mit einer Säure oder einer Base behandelt, oder

k) erwünschtenfalls ein erhaltenes Gemisch von Diastereoisomeren in die Racemate auftrennt, und/oder

l) erwünschtenfalls ein erhaltenes Racemat in die optischen Antipoden auftrennt, und/oder

m) erwünschtenfalls eine Carbonsäure der allgemeinen Formel I in ein Salz mit einer pharmazeutisch annehmbaren Base umwandelt.

Die Methode, die für die Cyclisierung einer Verbindung der allgemeinen Formel II, gemäß Verfahrensvariante a), verwendet wird, hängt von der Natur des Restes $R^4$ ab. Bedeutet $R^4$ Wasserstoff, so handelt es sich um eine Cyclisierung unter Wasserabspaltung. Diese kann nach an sich bekannten Methoden durchgeführt werden; beispielsweise mit Phosphorpentachlorid in einem inerten organischen Lösungsmittel, wie Dimethylformamid, bei niedrigen Temperaturen (z. B. bei etwa 0°C) und anschließendem Behandeln mit einer organischen Base, wie Pyridin, bei etwa Raumtemperatur. Bedeutet $R^4$ in einer Verbindung der allgemeinen Formel II $C_1-C_6$-Alkyl, so wird bei der Cyclisierung ein Alkohol der Formel $R^4-OH$ abgespalten. Diese Cyclisierungsmethode kann ebenfalls nach an sich bekannten Methoden durchgeführt werden; beispielsweise kann man eine Verbindung der Formel II in einer geeigneten organischen Säure, wie Essigsäure, erhitzen.

Verbindungen der Formel II, worin $R^4$ Wasserstoff bedeutet, verwendet man vorzugsweise in situ, d. h. ohne Isolierung aus dem Medium in dem sie vorher hergestellt wurden.

Die Reaktion der Verbindung der Formel III mit einem Anhydrid der allgemeinen Formel IV, gemäß Verfahrensvariante b), erfolgt beispielsweise durch Erhitzen der Reaktanden in Gegenwart einer Säure. In einer bevorzugten Ausführungsform verwendet man verdünnte Salzsäure und arbeitet bei der Siedetemperatur der Reaktionsmischung.

Die Reaktion einer Verbindung der allgemeinen Formel V mit einer Verbindung der allgemeinen Formel VIa, gemäß Verfahrensvariante c), wird unter oxydierenden Bedingungen durchgeführt. Geeignete Oxydationsmittel umfassen Bleitetraacetat, t-Butylhypochlorid oder dergleichen. Zweckmäßigerweise arbeitet man in einem inerten organischen Lösungsmittel. Geeignete Lösungsmittel sind beispielsweise aromatische Kohlenwasserstoffe, wie Benzol, Toluol oder dergleichen, halogenierte Kohlenwasserstoffe, wie Methylenchlorid, Chloroform, Chlorbenzol oder dergleichen, $C_1-C_6$-Dialkyl-Ketone, wie Aceton, Methyläthylketon oder dergleichen, Äther, wie Diäthyläther, Dioxan, Tetrahydrofuran oder dergleichen, Acetonitril, Essigester und dergleichen. Die Reaktion kann in einem Temperaturbereich von etwa −80°C bis zum Siedepunkt der Reaktionsmischung durchgeführt werden; vorzugsweise arbeitet man etwa bei Raumtemperatur.

Die Oxydation einer Verbindung der allgemeinen Formel XVIII, gemäß Verfahrensvariante d), kann nach an sich bekannten, bei Oxydationen von Alkoholen zu den entsprechenden Carbonsäuren üblichen Methoden durchgeführt werden (beispielsweie unter Verwendung eines chromhaltigen Oxydationsmittels).

Die Reaktion einer Verbindung der allgemeinen Formel VII mit einer Verbindung der allgemeinen Formel VIII, gemäß Verfahrensvariante e), wird vorzugsweise in einem inerten Lösungsmittel in Gegenwart einer Base durchgeführt. Verwendbare Basen sind beispielsweise Alkalimetallhydroxide, wie Natrium und Kaliumhydroxid, Alkalimetallhydride, wie Natrium- und Kaliumhydrid, Alkalimetallalkoxide, wie Natriummethoxid, Natriumäthoxid oder dergleichen, und Alkalimetallcarbonate, wie Natrium- und Kaliumcarbonat. Bedeutet $R^6$ in Verbindungen der allgemeinen Formel VIII

$C_2-C_6$-Alkanoyl oder Benzoyl, so sind $C_1-C_6$-Dialkylketone, wie Aceton, und Dimethylformamid oder, wenn ein Alkalimetallcarbonat als Base verwendet wird, eine Mischung aus Wasser und einem chlorierten Kohlenwasserstoff, wie Methylenchlorid, oder eine Mischung aus Wasser und Essigester geeignete Lösungsmittel. Bedeutet $R^6$ in einer Verbindung der allgemeinen Formel VIII Benzyl, so sind Wasser, Dimethylformamid und dergleichen geeignete Lösungsmittel. Zweckmäßigerweise kann man eine Verbindung der allgemeinen Formel VIII, worin $R^6$ $C_2-C_6$-Alkanoyl oder Benzoyl bedeutet, als Alkalimetallsalz, beispielsweise als Kaliumsalz, einsetzen und die Reaktion in Gegenwart einer katalytischen Menge eines Alkalimetalljodides, beispielsweise Kaliumjodid, durchführen. Die Reaktion einer Verbindung der allgemeinen Formel VII mit einer solchen der allgemeinen Formel VIII kann in einem Temperaturbereich von etwa 10°C bis Siedetemperatur der Reaktionsmischung durchgeführt werden. Vorzugsweise arbeitet man bei der Siedetemperatur der Reaktionsmischung.

Die Reaktion gemäß Verfahrensvariante f) kann nach an sich bekannten Methoden durchgeführt werden, wobei die verwendete Methode von der Natur der abzuspaltenden Gruppe abhängt. Man kann beispielsweise eine $C_2-C_6$-Alkanoyl- oder Benzoylgruppe mit einem wäßrigen Alkalimetallhydroxid, wie wäßrige Natronlauge oder wäßrige Kalilauge, mit wäßrigem Ammoniak oder mit einem $C_1-C_6$-Alkohol, wie Methanol, in Gegenwart des entsprechenden Alkalimetallalkoxids, wie Natriummethoxid, abspalten. Vorzugsweise verwendet man wäßriges Ammoniak. Eine Benzyl-Gruppe kann beispielsweise mit Natrium in flüssigem Ammoniak abgespalten werden.

Die Cyclisierung einer Verbindung der allgemeinen Formel IX unter Wasserabspaltung, gemäß Verfahrensvariante g), kann nach an sich bekannten Methoden durchgeführt werden; beispielsweise durch Behandeln mit Phosphorpentachlorid in einem. inerten organischen Lösungsmittel, wie Tetrahydrofuran, in einem Temperaturbereich von etwa 0°C bis Raumtemperatur. Verbindungen der allgemeinen Formel IX werden vorzugsweise in situ cyclisiert.

Gemäß Verfahrensvariante h) setzt man eine Verbindung der allgemeinen Formel X mit einer Verbindung der allgemeinen Formel VIII, worin $R^6$ $C_1-C_6$-Alkanoyl oder Benzoyl bedeutet, um. Diese Reaktion kann so durchgeführt werden, daß man eine Mischung dieser beiden Verbindungen bei Raumtemperatur stehen läßt.

Die Veresterung oder Amidierung einer Verbindung der allgemeinen Formel I, worin $R^2$ Hydroxy bedeutet, gemäß Verfahrensvariante i), kann nach an sich bekannten Methoden durchgeführt werden. Die Veresterung kann beispielsweise so durchgeführt werden, daß man eine Verbindung der allgemeinen Formel I, worin $R^2$ Hydroxy bedeutet, mit einem $C_1-C_6$-Alkohol, wie Methanol, Äthanol oder dergleichen, in Gegenwart einer geeigneten Säure, beispielsweise einer Mineralsäure, wie Salzsäure, oder mit einem geeigneten Diazoalkan, wie Diazomethan, behandelt. Andererseits kann man eine Verbindung der allgemeinen Formel I, worin $R^2$ Hydroxy bedeutet, in das entsprechende Säurechlorid überführen, beispielsweise durch Behandeln mit einem Chlorierungsmittel, wie Thionylchlorid, Phosphortrichlorid oder Phosphorpentachlorid, und dieses in an sich bekannter Weise mit einem $C_1-C_6$-Alkohol umsetzen. Einen t-Butylester kann man auch erhalten, indem man eine Carbonsäure der allgemeinen Formel I mit Isobuten in Gegenwart von Schwefelsäure umsetzt. Entsprechende Amide erhält man beispielsweise dadurch, daß man die obenerwähnten Säurechloride in üblicher Weise mit Ammoniak behandelt.

Gemäß Verfahrensvariante j) wird eine Verbindung der allgemeinen Formel I, worin $R^2$ $C_1-C_6$-Alkoxy bedeutet, in eine Verbindung der Formel I, worin $R^2$ Hydroxy bedeutet, übergeführt. Diese Reaktion kann nach an sich bekannten Methoden durchgeführt werden; beispielsweise durch Behandeln mit einem Alkalimetallhydroxid, wie Natrium- oder Kaliumhydroxid, zweckmäßigerweise in einem Temperaturbereich von etwa Raumtemperatur bis zum Siedepunkt der Reaktionsmischung, oder, wenn $R^2$ t-Butoxy bedeutet, durch Behandeln mit einer wasserfreien Säure.

Die Verbindungen der allgemeinen Formel I enthalten ein asymmetrisches Zentrum (1-Stellung) und können deshalb als optische Antipoden oder als Racemate vorliegen. Verbindungen der Formel I, die mehr als ein asymmetrisches Zentrum enthalten, können in verschiedenen diastereoisomeren Formen vorliegen. Die vorliegende Erfindung umfaßt sämtliche möglichen Stereoisomeren von Verbindungen der allgemeinen Formel I und alle möglichen diastereoisomeren Mischungen und Racemate, sowie die Auftrennung dieser diastereoisomeren Mischungen und die Auftrennung von Racematen, die nach an sich bekannten Methoden durchgeführt werden können.

Carbonsäuren der allgemeinen Formel I können mit pharmazeutisch annehmbaren Basen, gemäß Verfahrensvariante m), in Salze übergeführt werden. Demnach können beispielsweise Carbonsäuren der allgemeinen Formel I durch Behandeln mit Alkalimetallhydroxiden, wie Natriumhydroxid und Kaliumhydroxid, Erdalkalimetallhydroxiden, wie Calciumhydroxid und Magnesiumhydroxid, organischen Basen, wie Dicyclohexylamin, basischen Aminosäuren, wie Lysin und Arginin, in Salze übergeführt werden.

Die als Ausgangsstoffe verwendeten Verbindungen der allgemeinen Formel II sind neu und können hergestellt werden, indem man 1-Benzyloxycarbonyl-hexahydro-pyridazin-3-carbonsäure der Formel

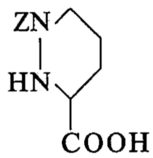

(XI)

worin Z Benzyloxycarbonyl bedeutet,
in einem entsprechenden $C_1$—$C_6$-Alkylester der allgemeinen Formel

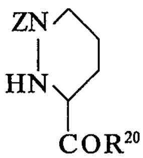

(XII)

worin $R^{20}$ und Z obige Bedeutung besitzen,
überführt und diesen $C_1$—$C_6$-Alkylester mit einem Säurechlorid der allgemeinen Formel

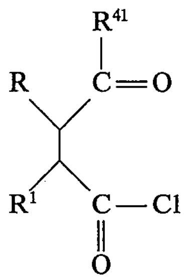

(XIII)

worin R und $R^1$ obige Bedeutung besitzen und $R^{41}$ $C_1$—$C_6$-Alkoxy oder Benzyloxy bedeutet, umsetzt, und aus der erhaltenen Verbindung der allgemeinen Formel

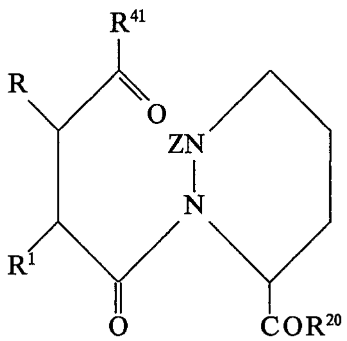

(XIV)

worin R, $R^1$, $R^{20}$, $R^{41}$ und Z obige Bedeutung besitzen, die mit Z bezeichnete Benzyloxycarbonylgruppe abspaltet.

Die Herstellung eines $C_1-C_6$-Alkylesters der allgemeinen Formel XII kann nach an sich bekannten, bei solchen Veresterungen allgemein üblichen Methoden, durchgeführt werden. Vorzugsweise stellt man mit Diazomethan den entsprechenden Methylester her.

Die Reaktion eines $C_1-C_6$-Alkylesters der allgemeinen Formel XII mit einem Säurechlorid der allgemeinen Formel XIII kann unter Schotten-Baumann-Bedingungen durchgeführt werden. Demnach kann man einen $C_1-C_6$-Alkylester, vorzugsweise den Methylester, der Formel XII etwa bei Raumtemperatur mit einem Säurechlorid der allgemeinen Formel XIII in einem inerten organischen Lösungsmittel, beispielsweise einem halogenierten Kohlenwasserstoff, wie Methylenchlorid, in Gegenwart einer verdünnten Natronlauge umsetzen.

Die Abspaltung der mit Z bezeichneten Benzyloxycarbonylgruppe aus einer Verbindung der Formel XIV erfolgt nach an sich bekannten Methoden. Zweckmäßigerweise verwendet man Bromwasserstoff in Eisessig und arbeitet bei Raumtemperatur. Bedeutet $R^{41}$ in einer Verbindung der Formel XIV Benzyloxy, so wird dieser Rest unter diesen Bedingungen ebenfalls abgespalten. Die Carbonsäurechloride der allgemeinen Formel XIII, sofern nicht vorbeschrieben, können wie in den nachfolgenden Beispielen beschrieben oder in analoger Weise dazu hergestellt werden.

Die weiter oben erwähnten Ausgangsstoffe der allgemeinen Formeln III, IV, VIa und VIII und die weiter unten erwähnten Ausgangsstoffe der allgemeinen Formel VIb sind bekannt oder können nach

an sich bekannten Methoden hergestellt werden.

Die weiter oben erwhnten Ausgangsstoffe der Formel V können beispielsweise hergestellt werden, indem man eine Verbindung der allgemeinen Formel IV mit Hydrazin behandelt.

Die Ausgangsstoffe der allgemeinen Formel XVIII sind neu und können hergestellt werden, indem man eine Verbindung der allgemeinen Formel V mit einer Verbindung der allgemeinen Formel

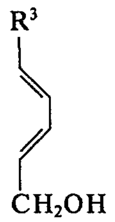

(VIb)

worin $R^3$ obige Bedeutung besitzt, umsetzt; und zwar in Analogie zu Verfahrensvariante c).

Die Ausgangsstoffe der allgemeinen Formel VII sind neu und können in Analogie zu den Verfahrensvarianten a), b) und c) hergestellt werden, indem man entsprechende Verbindungen der Formeln II, IV bzw. V als Ausgangsmaterial verwendet. Eine so erhaltene Verbindung der Formel VII kann, sofern in 2,3-Stellung eine Doppelbindung vorhanden ist, erwünschtenfalls katalytisch hydriert werden. Geeignete Katalysatoren sind z. B. Edelmetalle, wie Palladium, Platin, Ruthenium, Rhodium und Raney-Nickel, gegebenenfalls an einem geeigneten Trägermaterial gebunden (z. B. Palladium/Kohle, Rhodium/Aluminiumoxyd). Die katalytische Hydrierung kann in einem herkömmlichen inerten organischen Lösungsmittel, z. B. in einem aromatischen Kohlenwasserstoff, wie Benzol, Toluol, Xylol oder dergleichen, in einem $C_1-C_6$-Alkohol, wie Methanol, Äthanol oder dergleichen, oder in einem Äther, wie Dioxan oder dergleichen, durchgeführt werden. Vorteilhafterweise arbeitet man bei Raumtemperatur und Normaldruck.

Die Ausgangsstoffe der Formel IX sind neu und können hergestellt werden, indem man aus einer Verbindung der Formel XII die Benzyloxycarbonylgruppe abspaltet und die erhaltene Verbindung der allgemeinen Formel

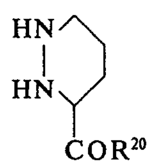

(XV)

worin $R^{20}$ obige Bedeutung besitzt, mit Itakonsäureanhydrid umsetzt, und den erhaltenen Stoff der allgemeinen Formel

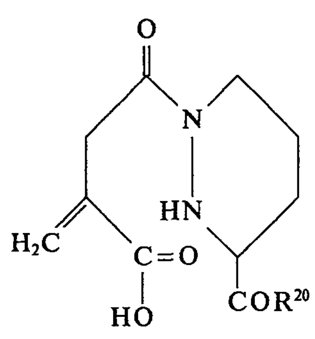

(XVI)

worin $R^{20}$ obige Bedeutung besitzt, mit einer Verbindung der obigen Formel VIII, worin $R^6$ $C_2-C_6$-Alkanoyl oder Benzoyl bedeutet, umsetzt.

Die Abspaltung der Benzyloxycarbonylgruppe aus einer Verbindung der Formel XII kann nach an sich bekannten Methoden durchgeführt werden; beispielsweise mit Wasserstoff in Gegenwart eines Katalysators, wie Palladium/Kohle.

Die Reaktion einer Verbindung der Formel XV mit Itakonsäureanhydrid wird zweckmäßigerweise in einem inerten organischen Lösungsmittel, z. B. in einem Äther, wie Dioxan, und etwa bei Raumtemperatur durchgeführt.

Die Reaktion einer Verbindung der Formel XVI mit einer solchen der Formel VIII, worin $R^6$ $C_2-C_6$-Alkanoyl oder Benzoyl bedeutet, kann in Analogie zu Verfahrensvariante h) durchgeführt werden.

Die Ausgangsstoffe der Formel X sind neu und können hergestellt werden, indem man eine Verbindung der allgemeinen Formel

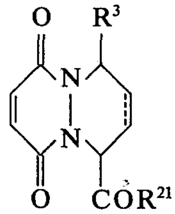

(XVII)

worin $R^3$, $R^{21}$ und die gestrichelte Linie obige Bedeutung besitzen,
mit einem Triarylphosphin umsetzt, und den erhaltenen Stoff mit Formaldehyd behandelt.

Für die obige Reaktion verwendet man vorzugsweise Triphenylphosphin und arbeitet nach an sich bekannten Methoden; z. B. bei Raumtemperatur in Gegenwart einer geeigneten Säure, wie Essigsäure.

Die Behandlung des Reaktionsproduktes mit Formaldehyd wird zweckmäßigerweise mit einer Lösung von Formaldehyd in Wasser, das wenig Methanol enthält, durchgeführt. Vorzugsweise arbeitet man bei Raumtemperatur.

Die Verbindungen der Formel XVII sind neu. Verbindungen der Formel XVII, die in der 2,3-Stellung eine Doppelbindung besitzen, können hergestellt werden, indem man 3,6-Dioxo-1,2,3,6-tetrahydropyridazin oder, was bevorzugt ist, ein Alkalimetallsalz davon, wie das Kaliumsalz, unter oxydierenden Bedingungen mit einer Verbindung der Formel VIa oder VIb umsetzt, und gegebenenfalls die Hydroxymethylgruppe in der 1-Stellung in an sich bekannter Weise in die Carboxygruppe überführt.

Die Reaktion von 3,6-Dioxo-1,2,3,6-tetrahydropyridazin oder von einem Alkalimetallsalz davon mit einer Verbindung der Formel VIa oder VIb kann in Analogie zu Verfahrensvariante c) durchgeführt werden. Die gegebenenfalls anschließende Oxydation der Hydroxymethylgruppe zur Carboxygruppe kann in Analogie zu Verfahrensvariante d) durchgeführt werden.

Verbindungen der Formel XVII, die in der 2,3-Stellung eine Einfachbindung besitzen, können hergestellt werden, indem man eine Verbindung der Formel XVII, die in der 2,3-Stellung eine Doppelbindung besitzt, katalytisch hydriert; und zwar in Analogie zur katalytischen Hydrierung einer Verbindung der Formel VII, die in der 2,3-Stellung eine Doppelbindung besitzt.

Eine Verbindung der Formel XVII, worin $R^{21}$ Hydroxy und $R^3$ Wasserstoff bedeutet, kann auch hergestellt werden, indem man Hexahydro-pyridazin-3-carbonsäure (Verbindung der Formel III) mit Maleinsäureanhydrid umsetzt. Diese Reaktion kann in Analogie zu Verfahrensvariante b) durchgeführt werden.

Verbindungen der allgemeinen Formel XVII, worin $R^{21}$ Hydroxy bedeutet, können in Analogie zu Verfahrensvariante j) aus Verbindungen der allgemeinen Formel XVII, worin $R^{21}$ $C_1-C_6$-Alkoxy bedeutet, erhalten werden.

Darüber hinaus können Verbindungen der allgemeinen Formel XVII, worin $R^{21}$ Hydroxy bedeutet, in Analogie zu Verfahrensvariante i) verestert werden, so daß man Verbindungen der allgemeinen Formel XVII erhält, worin $R^{21}$ $C_1-C_6$-Alkoxy bedeutet.

Die neuen erfindungsgemäßen Pyridazopyridazin-Derivate sind nützlich als antihypertensive Wirkstoffe. Sie hemmen das Angiotensin umwandelnde Enzym (ACE), das die Umwandlung von Angiotensin I zu Angiotensin II zustande bringt, und sind deshalb nützlich, um eine durch Angiotensin bewirkte erhöhte Muskelspannung zu vermindern oder zu lindern.

Die Aktivität der vorliegenden Pyridazopyridazin-Derivate, das das Angiotensin umwandelnde Enzym (ACE) in vitro zu hemmen, kann durch den folgenden Test bestimmt werden.

Die verwendete Methode basiert auf einer Methode von Cushman & Cheung (Biochem. Pharmacol. 20, 1637—1648) unter Berücksichtigung der Modifikationen von Hayakari et al. (Anal. Biochem. 84, 361—369). Das Substrat (Hippuryl-Histidyl-Leucin, 2 mM) wird mit oder ohne Testverbindung (verschiedene Konzentrationen) in Kaliumphosphat-Puffer (pH 8,3; 100 mM), das Natriumchlorid (300 mM) enthält, während 25 Minuten bei 37°C (Gesamtwert 500 µl) mit ACE inkubiert. Die Reaktion wird durch Zugabe von 3 ml Kaliumphosphat-Puffer (pH 8,3; 200 mM) bei 0°C abgebrochen. Man versetzt anschließend mit 2,4,6-Trichlor-s-triazin (3%) in 1,5 ml Dioxan und schüttelt die erhaltene Mischung, bis der gelbe Chromophor sich voll entwickelt hat. Die Proben werden anschließend zentrifugiert, um allenfalls entstandenen Festkörper zu entfernen. Der gelbe Chromophor, der durch Reaktion von 2,4,6-Trichlor-s-triazin mit freier Hippursäure entstanden ist, wird spektrophotometrisch bei 382 nm ausgemessen. Der $IC_{50}$-Wert ist diejenige Konzentration einer Testverbindung, die notwendig ist, um die Spaltung von Hippuryl-Histidyl-Leucin durch ACE unter den vorangehend beschriebenen Bedingungen um 50% zu reduzieren.

(rac.)-8-(Mercaptomethyl)-octahydro-6,9-dioxopyridazo[1,2-a]pyridazin-1-carbonsäure weist im vorhergehend beschriebenen Test eine $IC_{50}$(M) von $6,25 \times 10^{-8}$ auf.

Die Pyridazopyridazin-Derivate der allgemeinen Formel I können in Form von pharmazeutischen Präparaten, welche Verbindungen der allgemeinen Formel I zusammen mit geeigneten

pharmazeutischen Trägermaterialien enthalten, als Arzneimittel verwendet werden. Als Trägermaterialien kommen dabei für die enterale, beispielsweise orale, oder parenterale Verabreichung geeignete organische oder anorganische Trägermaterialien in Frage, beispielsweise Wasser, Gelatine, Gummi arabicum, Lactose, Stärke, Magnesiumstearat, Talk, pflanzliche Öle, Polyalkylenglykole, Vaselin und dergleichen. Die pharmazeutischen Präparate können in fester Form, beispielsweise als Tabletten, Dragées, Suppositorien oder Kapseln, oder in flüssiger Form, beispielsweise als Lösungen, Suspensionen oder Emulsionen, verarbeitet werden. Die pharmazeutischen Präparate können in üblichen pharmazeutischen Behandlungen, wie Sterilisation, unterworfen werden, und/oder Zusatzstoffe enthalten, wie Konservierungsmittel, Stabilisierungsmittel, Netzmittel oder Emulgiermittel, Salze, um den osmotischen Druck zu variieren, oder Puffer. Die pharmazeutischen Präparate können aber auch andere therapeutisch wertvolle Stoffe enthalten.

Die Pyridazopyridazin-Derivate, gemäß vorliegender Erfindung, können Erwachsenen in einer täglichen Dosis von etwa 0,1 – 100 mg, vorzugsweise ca. 1 – 50 mg, pro kg Körpergewicht verabreicht werden. Die tägliche Dosis kann in einer einzigen Dosis oder in verschiedenen Dosen verabreicht werden. Es sei an dieser Stelle betont, daß die oben angegebenen Dosierungen als Beispiele angegeben wurden, und daß sie, je nach Stärke der behandelten Symptome und Zustand der Patienten, nach oben oder nach unten variieren können und letztlich vom behandelnden Arzt festgelegt werden.

Die nachfolgenden Beispiele dienen der näheren Erläuterung der vorliegenden Erfindung.

## Beispiel 1

A) Eine Suspension von 18,0 g (0,068 Mol) (rac.)-Benzyloxycarbonyl-hexahydro-pyridazin-3-carbonsäure in 450 ml trockenem Diäthyläther wird mit einer ätherischen Diazomethanlösung (ca. 0,1 Mol) behandelt. Die erhaltene Mischung wird anschließend 2 Stunden bei Raumtemperatur gerührt, dreimal mit je 100 ml 10%iger Natriumcarbonatlösung und mit 100 ml Wasser gewaschen, über Magnesiumsulfat getrocknet und eingedampft. Man erhält 18,15 g (96%) (rac.)-1-Benzyloxycarbonyl-hexahydro-pyridazin-3-carbonsäuremethylester als bräunliches Öl.

B) 56 g (0,5 Mol) Itaconsäureanhydrid und 54 g (0,5 Mol) Benzylalkohol werden zusammen während 2 Stunden auf dem Dampfbad erwärmt. Die Mischung kristallisiert beim Abkühlen. Durch Umkristallisieren aus Toluol/Petroläther erhält man 74,22 g (67%) Monobenzylitaconat vom Schmelzpunkt 77 – 82°.

Eine Mischung aus 20 g (0,091 Mol) Monobenzylitaconat und 9,5 ml (0,13 Mol) Thioessigsäure wird während 3 Stunden unter Rückfluß zum Sieden erhitzt. Durch Abdampfen von überschüssiger Thioessigsäure erhält man 1-Benzyl-3-acetylthiomethyl-hydrogensuccinat.

Eine Lösung dieser Säure in trockenem Diäthyläther wird in einem Eisbad abgekühlt und mit Phosphorpentachlorid behandelt. Man erhält dabei das entsprechende Säurechlorid.

C) 15,99 g (0,0575 Mol) (rac.)-1-Benzyloxycarbonyl-hexahydro-pyridazin-3-carbonsäuremethylester [hergestellt nach Absatz (A)] werden in 350 ml Methylenchlorid aufgelöst. Bei Raumtemperatur versetzt man diese Lösung unter starkem Rühren über einen Zeitraum von 15 Minuten gleichzeitig mit einer Lösung des Säurechlorides [hergestellt nach Absatz (B)] in 130 ml Methylenchlorid und mit einer 0,5 M Natronlauge (137 ml; 0,068 Mol) (aus zwei verschiedenen Tropftrichtern). Nach beendeter Zugabe wird die Mischung während 18 Stunden bei Raumtemperatur gerührt. Nach Trennen der Phasen wird die organische Phase mit 200 ml gesättigter Natriumbicarbonatlösung gewaschen, über Magnesiumsulfat getrocknet und eingedampft, wobei 31,34 g braunes Öl anfallen. Dieses Öl wird an Kieselgel unter Eluieren mit Diäthyläther/Hexan (4 : 1) chromatographiert, wobei man beide Diastereoisomeren von (rac.)-1-Benzyl-3-methyl-2-[2-(Acetylthio)methyl]-3-(benzyloxycarbonyl)-propionyl-hexahydro-1,3-pyridazin-dicarboxylat erhält. Diastereoisomer A weist einen Rf-Wert von 0,6 und Diastereoisomer B einen Rf-Wert von 0,5 auf. Die Gesamtausbeute beträgt 20,21 g (63%).

D) 11,16 g (0,02 Mol) von Diastereoisomer A werden in 25 ml Eisessig aufgelöst. Diese Lösung wird mit 76 ml 45%igem Bromwasserstoff in Essigsäure behandelt und während 1,25 Stunden bei Raumtemperatur stehengelassen. Nach Eindampfen wird der Rückstand in 125 ml Dimethylformamid aufgenommen, auf 0° abgekühlt und mit 4,47 g (0,021 Mol) Phosphorpentachlorid versetzt. Die Mischung wird während 30 Minuten bei 0° und anschließend während 2 Stunden bei Raumtemperatur gerührt. Nach Zugabe von 23 ml Pyridin wird die Mischung während weiterer 2 Stunden gerührt. Nach Eindampfen der erhaltenen Lösung wird der Rückstand in 600 ml Essigester gelöst, und diese Lösung nacheinander mit 110 ml 2 M Salzsäure, 200 ml gesättigter Natriumbicarbonatlösung und 150 ml gesättigter Kochsalzlösung gewaschen. Die organische Phase wird über Magnesiumsulfat getrocknet und eingedampft, wobei 7,36 g braunes Öl anfallen. Säulenchromatographie an Kieselgel unter Eluieren mit Chloroform/Essigester (1 : 1) ergibt 1,15 g (18%) (rac.)-Methyl-8-(acetylthio)methyl-octahydro-6,9-dioxopyridazo[1,2-a]pyridazin-1-carboxylat (Diastereoisomer A) vom Schmelzpunkt 84 – 86° (Essigester/Hexan).

In analoger Weise erhält man aus dem Diastereoisomer B [hergestellt nach Absatz (B)] in 16%iger

Ausbeute (rac.)-Methyl-8-(acetylthio)methyl-octahydro-6,9-dioxopyridazo[1,2-a]pyridazin-1-carboxylat (Diastereoisomer B) vom Schmelzpunkt 130—132° (Essigester/Hexan).

## Beispiel 2

Eine Lösung von 0,84 g (2,67 mMol) (rac.)-Methyl-8-(acetylthio)methyl-octahydro-6,9-dioxopyridazo-[1,2-a]pyridazin-1-carboxylat [Diastereoisomer A; siehe Beispiel 1, Absatz (D)] in einer Mischung aus 5 ml Methanol und 10 ml 1 M Natronlauge wird unter einer Stickstoffatmosphäre während 2 Stunden bei Raumtemperatur gerührt, anschließend mit Wasser verdünnt und mit Chloroform ausgeschüttelt. Die wäßrige Phase wird mit konzentrierter Salzsäure sauer gestellt, mit Natriumchlorid gesättigt und mit Chloroform ausgeschüttelt. Die vereinigten Chloroformauszüge werden über Magnesiumsulfat getrocknet und eingedampft. Durch Umkristallisieren des farblosen Rückstandes (0,74 g) aus Essigester/Hexan erhält man 0,36 g (52%) (rac.)-8-(Mercaptomethyl)-octahydro-6,9-dioxopyridazo[1,2-a]pyridazin-1-carbonsäure (Diastereoisomer A) vom Schmelzpunkt 195—196°.

## Beispiel 3

A) Eine Lösung von 3,2 g (rac.)-1-Benzyloxycarbonyl-hexahydro-pyridazin-3-carbonsäure-tert.Butylester in 30 ml Methanol wird über 320 mg Palladium/Kohle (5%) hydriert. Nach Abfiltrieren des Katalysators wird das Filtrat zur Trockne eingedampft. Der erhaltene rohe (rac.)-Hexahydropyridazin-3-carbonsäure-tert.Butylester wird in 30 ml Dioxan aufgenommen, auf 0° abgekühlt und mit einer Lösung von 1,12 g Itaconsäureanhydrid in 10 ml Dioxan behandelt. Man rührt während 4 Stunden bei Raumtemperatur, entfernt das Lösungsmittel, und verteilt den Rückstand zwischen Chloroform und gesättigter Natriumbicarbonatlösung. Die wäßrige Phase wird mit Salzsäure auf pH 2 eingestellt und mit Chloroform ausgeschüttelt. Die Chloroformauszüge werden über Magnesiumsulfat getrocknet und eingedampft. Aus dem Rückstand erhält man durch Umkristallisieren aus Essigester 1,73 g (57%) (rac.)-3-tert.Butoxycarbonyl-hexahydro-$\alpha$-methylen-$\gamma$-oxo-1-pyridazin-buttersäure als weiße Kristalle vom Schmelzpunkt 153—154°.

B) 5,5 g (rac.)-3-tert.Butoxycarbonyl-hexahydro-$\alpha$-methylen-$\gamma$-oxo-1-pyridazin-buttersäure werden in 18 ml Thioessigsäure während 3 Tagen bei Raumtemperatur gerührt. Nach Entfernen von überschüssiger Thioessigsäure im Vakuum wird der Rückstand in 220 ml Tetrahydrofuran aufgenommen. Nach Abkühlen auf 0° versetzt man mit 3,85 g Phosphorpentachlorid und rührt die Mischung während einer halben Stunde bei 0° und während 3 Stunden bei Raumtemperatur. Anschließend entfernt man das Lösungsmittel und verteilt den Rückstand zwischen Chloroform und gesättigter Natriumbicarbonatlösung. Die Chloroformphase wird mit halbgesättigter Kochsalzlösung gewaschen, über Magnesiumsulfat getrocknet und eingedampft. Den Rückstand chromatographiert man an Kieselgel unter Eluieren mit Chloroform/Essigester (3 : 1) und erhält dabei 4,05 g (62%) (rac.)-tert.Butyl-8-(acetylthio)methyl-octahydro-6,9-dioxopyridazo[1,2-a]pyridazin-1-carboxylat als ölige Mischung von Diastereoisomeren. Durch Kristallisieren aus Essigester/Hexan erhält man Diastereoisomer B als weißen Festkörper vom Schmelzpunkt 124—128°. Durch Eindampfen der Mutterlauge erhält man ein Öl, das vorwiegend Diastereoisomer A enthält.

C) (i) 2,47 g rohes (rac.)-tert.Butyl-8-(acetylthio)methyl-octahydro-6,9-dioxopyridazo[1,2-a]pyridazin-1-carboxylat (Diastereoisomer A) werden während 1,5 Stunden mit 25 ml frisch destillierter Trifluoressigsäure bei Raumtemperatur gerührt. Man dampft die Reaktionsmischung ein, versetzt den Rückstand mit Toluol und dampft erneut ein. Durch Umkristallisieren aus Essigester/Petroläther (60—80°) erhält man 1,57 g (75%) (rac.)-8-(Acetylthio)methyl-octahydro-6,9-dioxopyridazo[1,2-a]pyridazin-1-carbonsäure (Diastereoisomer A) als weißen Festkörper vom Schmelzpunkt 148—149°.

C) (ii) In Analogie zum vorangehenden Absatz erhält man aus 1,29 g (rac.)-tert.Butyl-8-(acetylthio)methyl-octahydro-6,9-dioxopyridazo[1,2-a]pyridazin-1-carboxylat (Diastereoisomer B) 0,75 g (69%) (rac.)-8-(Acetylthio)methyl-octahydro-6,9-dioxopyridazo[1,2-a]pyridazin-1-carbonsäure (Diastereoisomer B) als weißen Festkörper vom Schmelzpunkt 206—107°.

D) (i) 0,08 g (rac.)-8-(Acetylthio)methyl-octahydro-6,9-dioxopyridazo[1,2-a]pyridazin-1-carbonsäure (Diastereoisomer A) wird unter einer Stickstoffatmosphäre mit einer Mischung aus 1 ml Wasser und 1 ml konz. wäßrigem Ammoniak während 1 Stunde bei Raumtemperatur gerührt. Nach Ansäuren auf pH 1 wird die Lösung mit Kochsalz gesättigt und mit Chloroform ausgeschüttelt. Die Chloroformauszüge werden über Magnesiumsulfat getrocknet und eingedampft. Durch Kristallisieren des Rückstandes aus Essigester/Hexan erhält man 0,05 g (73%) (rac.)-8-(Mercaptomethyl)-octahydro-6,9-dioxopyridazo[1,2-a]pyridazin-1-carbonsäure (Diastereoisomer A) als weißen Festkörper vom Schmelzpunkt 194—196°.

D) (ii) In Analogie zum vorangehenden Absatz erhält man aus (rac.)-8-(Acetylthio)methyl-octahydro-6,9-dioxopyridazo[1,2-a]pyridazin-1-carbonsäure (Diastereoisomer B) (rac.)-8-(Mercaptomethyl)-octahydro-6,9-dioxopyridazo[1,2-a]pyridazin-1-carbonsäure (Diastereoisomer B) als weißen Festkörper vom Schmelzpunkt 162—166°.

## Beispiel 4

A) 163 g 3,6-Dioxo-1,2,3,6-tetrahydropyridazin-kaliumsalz und 122 g Methyl-penta-2,4-dienoat werden unter einer Stickstoffatmosphäre mit 2,7 l Acetonitril bei −15° gerührt. Über einen Zeitraum von 35 Minuten versetzt man mit 130 ml tert.Butylhypochlorit. Die erhaltene gelbe Suspension wird bei −15° während 2 Stunden gerührt und anschließend bei Raumtemperatur über Nacht stehen gelassen. Nach Abfiltrieren des braunen Festkörpers unter Nachwaschen mit 200 ml Acetonitril wird eingedampft. Der Rückstand wird zwischen Methylenchlorid und Wasser verteilt. Die organische Phase trocknet man über Magnesiumsulfat und dampft ein. Der Rückstand wird mit Hexan ausgewaschen und aus Essigester umkristallisiert. Man erhält 84,1 g (35%) (rac.)-Methyl-1,4,6,9-tetrahydro-6,9-dioxopyridazo[1,2-a]pyridazin-1-carboxylat als schwach gelben Festkörper vom Schmelzpunkt 143−145°.

B) 6 g (rac.)-Methyl-1,4,6,9-tetrahydro-6,9-dioxopyridazo[1,2-a]pyridazin-1-carboxylat werden in 70 ml Essigsäure aufgelöst. Die Lösung wird mit 7,8 g Triphenylphosphin versetzt, während 2 Stunden bei Raumtemperatur gerührt und anschließend zur Trockne eingedampft. Den Rückstand versetzt man mit 11 ml 40%iger Formalinlösung und rührt die erhaltene Mischung während 3 Stunden bei Raumtemperatur. Man dampft zur Trockne ein, nimmt den Rückstand in 70 ml Thioessigsäure auf und läßt während 3 Tagen bei Raumtemperatur stehen. Nach erneutem Eindampfen wird der Rückstand chromatographiert, dabei erhält man 1,12 g (13%) (rac.)-Methyl-8-(acetylthio)methyl-1,4,6,7,8,9-hexahydro-6,9-dioxopyridazo[1,2-a]pyridazin-1-carboxylat als weißen Festkörper vom Schmelzpunkt 135−136° (Essigester/Hexan).

## Beispiel 5

A) Eine Mischung aus 76,7 g (rac.)-Methyl-1,4,6,9-tetrahydro-6,9-dioxopyridazo[1,2-a]pyridazin-1-carboxylat und 350 ml 2N Salzsäure wird während 0,75 Stunden unter Rückfluß zum Sieden erhitzt. Die Mischung wird auf 0° abgekühlt und filtriert. Durch Umkristallisieren des Rückstandes aus Wasser erhält man 55,15 g (71%) (rac.)-1,4,6,9-Tetrahydro-6,9-dioxopyridazo[1,2-a]pyridazin-1-carbonsäure-monohydrat als weißen Festkörper vom Schmelzpunkt 185−187° (Zersetzung).

B) Eine Mischung aus 6,24 g (rac.)-1,4,6,9-Tetrahydro-6,9-dioxopyridazo[1,2-a]pyridazin-1-carbonsäure, 8,64 g Triphenylphosphin und 150 ml Essigsäure wird während kurzer Zeit erwärmt. Anschließend rührt man während 2,5 Stunden bei Raumtemperatur und dampft ein. Den Rückstand behandelt man während 3,5 Stunden bei Raumtemperatur mit 11 ml 40%iger Formalinlösung und dampft die Mischung anschließend ein. Nach Aufnehmen des Rückstandes in 80 ml Thioessigsäure läßt man die Lösung während 3 Tagen bei Raumtemperatur stehen. Man dampft ein und verteilt den Rückstand zwischen Chloroform und gesättigter Natriumbicarbonatlösung. Die wäßrige Phase wird mit verdünnter Salzsäure sauer gestellt und mit Chloroform ausgeschüttelt. Die Chloroformauszüge werden über Magnesiumsulfat getrocknet und eingedampft. Der ölige Rückstand wird in Dioxan suspendiert und mit ätherischer Diazomethanlösung bis zur bleibenden Gelbfärbung behandelt. Überschüssiges Diazomethan wird mit Essigsäure zerstört. Nach Eindampfen wird der Rückstand in Essigester aufgenommen, und nacheinander mit gesättigter Natriumbicarbonatlösung und halbgesättigter Kochsalzlösung gewaschen. Man trocknet die organische Phase über Magnesiumsulfat und dampft ein. Das verbleibende Öl chromatographiert man an Kieselgel und erhält 1,95 g (23%) (rac.)-Methyl-8-(acetylthio)methyl-1,4,6,7,8,9-hexahydro-6,9-dioxopyridazo[1,2-a]pyridazin-1-carboxylat vom Schmelzpunkt 132−133° (Essigester/Hexan).

C) In Analogie zu den Angaben in Beispiel 2 erhält man aus 0,94 g (rac.)-Methyl-8-(acetylthio)methyl-1,4,6,7,8,9-hexahydro-6,9-dioxopyridazo[1,2-a]pyridazin-1-carboxylat nach Umkristallisieren aus Essigester/Toluol 0,32 g (41%) (rac.)-8-(Mercaptomethyl)-1,4,6,7,8,9-hexahydro-6,9-dioxopyridazo[1,2-a]pyridazin-1-carbonsäure vom Schmelzpunkt 155−162° (Zersetzung).

## Beispiel 6

A) (i) 2 g (rac.)-Methyl-1,4,6,9-tetrahydro-6,9-dioxopyridazo[1,2-a]pyridazin-1-carboxylat werden in 50 ml Methanol über 200 mg Palladium/Kohle (10%) während 2 Stunden bei Raumtemperatur und Normaldruck hydriert. Nach Filtrieren und Eindampfen der Lösung wird der Rückstand aus Essigester umkristallisiert. Man erhält 0,75 g (37%) (rac.)-Methyl-1,2,3,4,6,9-hexahydro-6,9-dioxo-pyridazo[1,2-a]-pyridazin-1-carboxylat als weißen Festkörper vom Schmelzpunkt 121°.

A) (ii) Eine Lösung von 1,20 g (rac.)-Hexahydro-pyridazin-3-carbonsäure in einer Mischung aus 23 ml Wasser, 3 ml konzentrierter Salzsäure und 1,5 ml Methanol wird mit 0,90 g Maleinsäureanhydrid versetzt und während 12 Stunden unter Rückfluß zum Sieden erhitzt. Man dampft zur Trockne ein, und löst den Rückstand in 40 ml gesättigter methanolischer Salzsäure. Die Lösung wird während 2,5 Stunden zum Rückfluß erhitzt und anschließend eingedampft. Das erhaltene braune Öl chromatographiert man an Kieselgel und erhält dabei 0,63 g (30%) (rac.)-Methyl-1,2,3,4,6,9-hexahy-

dro-6,9-dioxopyridazo[1,2-a]pyridazin-1-carboxylat, das, nach Umkristallisieren aus Chloroform/Hexan, bei 121,5° schmilzt.

B) In Analogie zu den Angaben in Beispiel 4 (B) erhält man aus 0,224 g (rac.)-Methyl-1,2,3,4,6,9-hexahydro-6,9-dioxopyridazo[1,2-a]pyridazin-1-carboxylat nach Umkristallisieren aus Essigester/Hexan 0,04 g (13%) (rac.)-Methyl-8-(acetylthio)methyl-octahydro-6,9-dioxopyridazo[1,2-a]pyridazin-1-carboxylat (Diastereoisomer B) als weißen Festkörper vom Schmelzpunkt 128—132°.

## Beispiel 7

A) Eine Suspension von 49,5 g (rac.)-1,4,6,9-Tetrahydro-6,9-dioxopyridazo[1,2-a]pyridazin-1-carbonsäure in 1 l siedendem Äthanol wird mit einer Lösung von 26,6 g (+)-1-Phenyläthylamin in 200 ml Äthanol versetzt. Man läßt bei 0° stehen und filtriert anschließend den ausgefallenen kristallinen Festkörper ab. Durch Umkristallisieren aus Äthanol (bis zum konstanten Drehwert) erhält man 26,5 g (+)-1-Phenyläthylammoniumsalz der obigen Carbonsäure; $[\alpha]^{20}_{436} = +898°$ (c=0,5% in Methanol). Dieses Salz wird in 1 l 50%igem Methanol gelöst und mit einem Sulfonsäure-Ionenaustauscher gerührt. Nach Filtrieren unter Nachwaschen mit 50%igem Methanol wird eingedampft. Der Rückstand kristallisiert aus Wasser und ergibt 15,3 g (+)-1,4,6,9-Tetrahydro-6,9-dioxo-pyridazo[1,2-a]pyridazin-1-carbonsäure-hydrat; $[\alpha]^{20}_{436} = +1043°$ (c=0,5% in Wasser).

Auf die gleiche Weise erhält man mit (—)-1-Phenyläthylamin(—)-1,4,6,9-tetrahydro-6,9-dioxopyridazo[1,2-a]pyridazin-1-carbonsäure-hydrat; $[\alpha]^{20}_{436} = -1046°$ (c=0,5% in Wasser).

B) In Analogie zu den Angaben in Beispiel 5 (B) erhält man aus (+)-1,4,6,9-Tetrahydro-6,9-dioxopyridazo[1,2-a]pyridazin-1-carbonsäure (+)-Methyl-8-(acetylthio)methyl-1,4,6,7,8,9-hexahydro-6,9-dioxopyridazo[1,2-a]pyridazin-1-carboxylat [Schmelzpunkt 114—115°; $[\alpha]^{20}_{436} = +1486°$ (c=0,5% in Methanol)] und aus (—)-1,4,6,9-Tetrahydro-6,9-dioxopyridazo[1,2-a]pyridazin-1-carbonsäure erhält man (—)-Methyl-8-(acetylthio)methyl-1,4,6,7,8,9-hexahydro-6,9-dioxopyridazo[1,2-a]pyridazin-1-carboxylat vom Schmelzpunkt 114—115°; $[\alpha]^{20}_{436} = -1438°$ (c=0,5% in Methanol).

C) Aus 1,18 g (—)-Methyl-8-(acetylthio)methyl-1,4,6,7,8,9-hexahydro-6,9-dioxopyridazo[1,2-a]pyridazin-1-carboxylat erhält man in Analogie zu den Angaben in Beispiel 2 nach Umkristallisieren aus Essigester/Toluol 0,46 g (48%) (—)-8-(Mercaptomethyl)-1,4,6,7,8,9-hexahydro-6,9-dioxopyridazo-[1,2-a]pyridazin-1-carbonsäure vom Schmelzpunkt 139—146° (Zersetzung); $[\alpha]^{20}_{365} = -1779°$ (c=0,5% in Methanol).

Die folgenden Beispiele beschreiben pharmazeutische Präparate, enthaltend ein Pyridazopyridazin-Derivat gemäß vorliegender Erfindung:

## Beispiel A

In bekannter Weise werden Tabletten folgender Zusammensetzung hergestellt:

| Bestandteile | mg/Tablette |
|---|---|
| Pyridazopyridazin-Derivat | 10,0 |
| Lactose | 125,0 |
| Maisstärke | 75,0 |
| Talk | 4,0 |
| Magnesiumstearat | 1,0 |
| Total | 215,0 |

## Beispiel B

In bekannter Weise werden Kapseln folgender Zusammensetzung hergestellt:

| Bestandteile | mg/Kapsel |
|---|---|
| Pyridazopyridazin-Derivat | 25,0 |
| Lactose | 150,0 |
| Maisstärke | 20,0 |
| Talk | 5,0 |
| Total | 200,0 |

**Patentansprüche für die Vertragsstaaten: BE, CH, DE, FR, GB, IT, LI, LU, NL, SE**

1. Pyridazopyridazin-Derivate der allgemeinen Formel

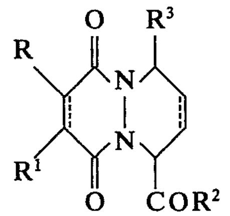

(I)

worin einer der Reste R und $R^1$ Wasserstoff oder $C_1-C_6$-Alkyl und der andere einen Rest der Formel

$$-(A)_n-Y \qquad (i)$$

bedeutet, wobei A eine gegebenenfalls durch $C_1-C_6$-Alkyl substituierte Methylen-, Äthylen- oder Propylengruppe, Y Mercapto, $C_2-C_6$-Alkanoylthio, Benzoylthio oder Benzylthio und n die Zahl 0 oder 1 bedeuten, $R^2$ Hydroxy, $C_1-C_6$-Alkoxy oder Amino, $R^3$ Wasserstoff, $C_1-C_6$-Alkyl oder gegebenenfalls durch $C_1-C_6$-Alkyl, $C_1-C_6$-Alkoxy, Halogen oder Trifluormethyl substituiertes Phenyl und die gestrichelten Linien fakultative Bindungen bedeuten,
und Salze von Carbonsäuren der allgemeinen Formel I mit pharmazeutisch annehmbaren Basen.

2. Pyridazopyridazin-Derivate gemäß Anspruch 1, worin R Wasserstoff, $R^1$ einen Rest der Formel (i), $R^2$ Hydroxy oder $C_1-C_6$-Alkoxy und $R^3$ Wasserstoff bedeuten und die in 7,8-Stellung eine Einfachbindung besitzen.

3. Pyridazopyridazin-Derivate gemäß Anspruch 2, worin der Rest der Formel (i) Mercaptomethyl oder $C_2-C_6$-Alkanoylthiomethyl bedeutet.

4. (rac.)-Methyl-8-(acetylthio)methyl-octahydro-6,9-dioxopyridazo[1,2-a]pyridazin-1-carboxylat.

5. (rac.)-8-(Mercaptomethyl)-octahydro-6,9-dioxopyridazo[1,2-a]pyridazin-1-carbonsäure.

6. Pyridazopyridazin-Derivate gemäß einem der Ansprüche 1–5 und gegebenenfalls ihre Salze mit pharmazeutisch annehmbaren Basen zur Verwendung als pharmazeutische Wirkstoffe.

7. Pyridazopyridazin-Derivate gemäß einem der Ansprüche 1–5 und gegebenenfalls ihre Salze mit pharmazeutisch annehmbaren Basen zur Verwendung als Antihypertensiva.

8. Verfahren zur Herstellung von Pyridazopyridazin-Derivaten der in Anspruch 1 definierten allgemeinen Formel I und ihrer Salze mit pharmazeutisch annehmbaren Basen, dadurch gekennzeichnet, daß man

a) zur Herstellung einer Verbindung der allgemeinen Formel I, worin $R^2$ $C_1-C_6$-Alkoxy und $R^3$ Wasserstoff bedeuten und die in der 2,3- und der 7,8-Stellung Einfachbindungen besitzt, eine Verbindung der allgemeinen Formel

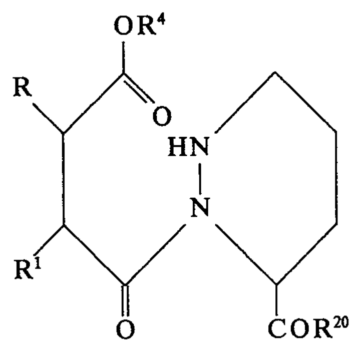

(II)

worin R und $R^1$ die in Anspruch 1 angegebene Bedeutung besitzen, $R^4$ Wasserstoff oder $C_1-C_6$-Alkyl und $R^{20}$ $C_1-C_6$-Alkoxy bedeuten,
cyclisiert, oder

b) zur Herstellung einer Verbindung der allgemeinen Formel I, worin $R^2$ Hydroxy und $R^3$ Wasserstoff bedeuten und die in der 2,3-Stellung eine Einfachbindung besitzt, die Verbindung der Formel

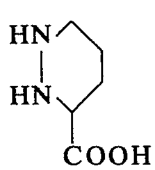

(III)

mit einem Anhydrid der allgemeinen Formel

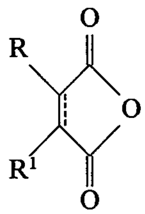

(IV)

worin R, $R^1$ und die gestrichelte Linie die in Anspruch 1 angegebene Bedeutung besitzen, umsetzt, oder

c) zur Herstellung einer Verbindung der allgemeinen Formel I, die in der 2,3-Stellung eine Doppelbindung besitzt, eine Verbindung der allgemeinen Formel

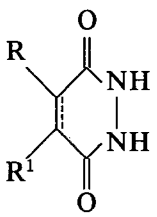

(V)

worin R, $R^1$ und die gestrichelte Linie die in Anspruch 1 angegebene Bedeutung besitzen, unter oxydierenden Bedingungen mit einer Verbindung der allgemeinen Formel

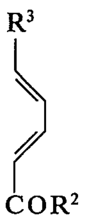

(VIa)

worin $R^2$ und $R^3$ die in Anspruch 1 angegebene Bedeutung besitzen, umsetzt, oder

d) zur Herstellung einer Verbindung der allgemeinen Formel I, worin $R^2$ Hydroxy bedeutet und die in der 2,3-Stellung eine Doppelbindung besitzt, eine Verbindung der allgemeinen Formel

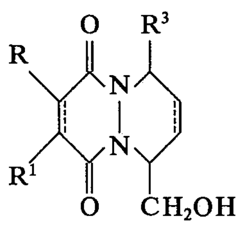

(XVIII)

worin R, $R^1$, $R^3$ und die gestrichelten Linien die in Anspruch 1 angegebene Bedeutung besitzen, oxydiert, oder

e) zur Herstellung einer Verbindung der allgemeinen Formel I, worin Y im Rest der Formel (i) $C_2-C_6$-Alkanoylthio, Benzoylthio oder Benzylthio bedeutet, eine Verbindung der allgemeinen Formel

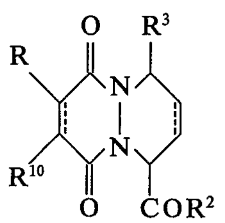

(VII)

worin $R^2$, $R^3$ und die gestrichelten Linien die in Anspruch 1 angegebene Bedeutung besitzen und einer der Reste R und $R^{10}$ Wasserstoff oder $C_1-C_6$-Alkyl und der andere einen Rest der Formel

$$-(A)_n-X \qquad \text{(ii)}$$

bedeutet, wobei A und n die in Anspruch 1 angegebene Bedeutung besitzen und X Halogen bedeutet, mit einer Verbindung der allgemeinen Formel

$$R^6-SH \qquad (VIII)$$

worin $R^6$ $C_2-C_6$-Alkanoyl, Benzoyl oder Benzyl bedeutet, umsetzt, oder

f) zur Herstellung einer Verbindung der allgemeinen Formel I, worin Y Mercapto bedeutet, in einer Verbindung der allgemeinen Formel I, worin Y $C_2-C_6$-Alkanoylthio, Benzoylthio oder Benzylthio bedeutet, die $C_2-C_6$-Alkanoyl-, Benzoyl- oder Benzyl-Gruppe abspaltet, oder

g) zur Herstellung einer Verbindung der allgemeinen Formel I, worin R Wasserstoff und $R^1$ einen Rest der Formel (i) bedeutet, wobei A eine Methylengruppe, Y $C_2-C_6$-Alkanoylthio oder Benzoylthio und n die Zahl 1 bedeuten, $R^2$ $C_1-C_6$-Alkoxy und $R^3$ Wasserstoff bedeuten, und die in der 2,3- und der 7,8-Stellung Einfachbindungen besitzt, eine Verbindung der allgemeinen Formel

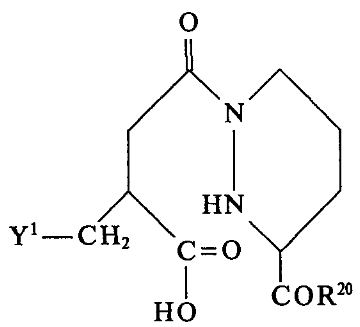

(IX)

worin $R^{20}$ obige Bedeutung besitzt und $Y^1$ $C_2-C_6$-Alkanoylthio oder Benzoylthio bedeutet, cyclisiert, oder

h) zur Herstellung einer Verbindung der allgemeinen Formel I, worin R Wasserstoff und $R^1$ einen Rest der Formel (i) bedeuten, wobei A eine Methylengruppe, Y $C_2-C_6$-Alkanoylthio oder Benzoylthio und n die Zahl 1 bedeuten, und $R^2$ Hydroxy oder $C_1-C_6$-Alkoxy bedeutet und die in der 7,8-Stellung eine Einfachbindung besitzt, eine Verbindung der allgemeinen Formel

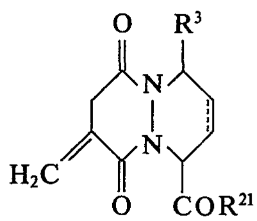

(X)

worin $R^3$ und die gestrichelte Linie die in Anspruch 1 angegebene Bedeutung besitzen und $R^{21}$ Hydroxy oder $C_1-C_6$-Alkoxy bedeutet, mit einer Verbindung der Formel VIII, worin $R^6$ $C_2-C_6$-Alkanoyl oder Benzoyl bedeutet, umsetzt, oder

i) zur Herstellung einer Verbindung der allgemeinen Formel I, worin $R^2$ $C_1-C_6$-Alkoxy oder Amino bedeutet, eine entsprechende Verbindung der allgemeinen Formel I, worin $R^2$ Hydroxy bedeutet, verestert oder amidiert, oder

j) zur Herstellung einer Verbindung der allgemeinen Formel I, worin $R^2$ Hydroxy bedeutet, eine entsprechende Verbindung der allgemeinen Formel I, worin $R^2$ $C_1-C_6$-Alkoxy bedeutet, mit einer Säure oder einer Base behandelt, oder

k) erwünschtenfalls ein erhaltenes Gemisch von Diastereoisomeren in die Racemate auftrennt, und/oder

l) erwünschtenfalls ein erhaltenes Racemat in die optischen Antipoden auftrennt, und/oder

m) erwünschtenfalls eine Carbonsäure der allgemeinen Formel I in ein Salz mit einer pharmazeutisch annehmbaren Base umwandelt.

9. Verfahren gemäß Anspruch 8, dadurch gekennzeichnet, daß man nach den Verfahrensvarianten a), b), c), d), e), f), i) oder j) verfährt und erforderlichenfalls noch die Varianten k), l) oder m) anschließt.

10. Arzneimittel, enthaltend ein Pyridazopyridazin-Derivat gemäß einem der Ansprüche 1–5 oder gegebenenfalls ein Salz davon mit einer pharmazeutisch annehmbaren Base.

11. Antihypertensivum, enthaltend ein Pyridazopyridazin-Derivat gemäß einem der Ansprüche 1–5 oder gegebenenfalls ein Salz davon mit einer pharmazeutisch annehmbaren Base.

**Patentansprüche für den Vertragsstaat: AT**

1. Verfahren zur Herstellung von Pyridazopyridazin-Derivaten der allgemeinen Formel

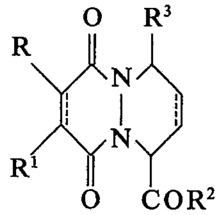

(I)

worin einer der Reste R und $R^1$ Wasserstoff oder $C_1-C_6$-Alkyl und der andere einen Rest der Formel

$$-(A)_n-Y \quad \text{(i)}$$

bedeutet, wobei A eine gegebenenfalls durch $C_1-C_6$-Alkyl substituierte Methylen-, Äthylen- oder Propylengruppe, Y Mercapto, $C_2-C_6$-Alkanoylthio, Benzoylthio oder Benzylthio und n die Zahl 0 oder 1 bedeuten, $R^2$ Hydroxy, $C_1-C_6$-Alkoxy oder Amino, $R^3$ Wasserstoff, $C_1-C_6$-Alkyl oder gegebenenfalls durch $C_1-C_6$-Alkyl, $C_1-C_6$-Alkoxy, Halogen oder Trifluormethyl substituiertes Phenyl und die gestrichelten Linien fakultative Bindungen bedeuten,
und Salzen von Carbonsäuren der allgemeinen Formel I mit pharmazeutisch annehmbaren Basen, dadurch gekennzeichnet, daß man

a) zur Herstellung einer Verbindung der allgemeinen Formel I, worin $R^2$ $C_1-C_6$-Alkoxy und $R^3$ Wasserstoff bedeuten und die in der 2,3- und der 7,8-Stellung Einfachbindungen besitzt, eine Verbindung der allgemeinen Formel

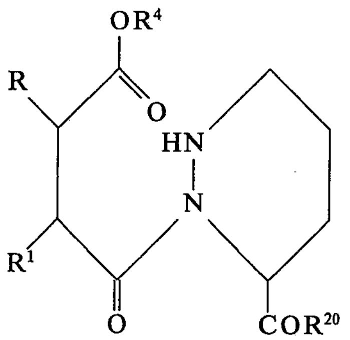

(II)

worin R und $R^1$ die oben angegebene Bedeutung besitzen, $R^4$ Wasserstoff oder $C_1-C_6$-Alkyl und $R^{20}$ $C_1-C_6$-Alkoxy bedeuten,
cyclisiert, oder

b) zur Herstellung einer Verbindung der allgemeinen Formel I, worin $R^2$ Hydroxy und $R^3$ Wasserstoff bedeuten und die in der 2,3-Stellung eine Einfachbindung besitzt, die Verbindung der Formel

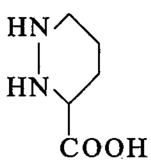

(III)

mit einem Anhydrid der allgemeinen Formel

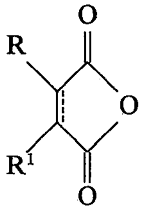

(IV)

worin R, $R^1$ und die gestrichelte Linie die oben angegebene Bedeutung besitzen,
umsetzt, oder

c) zur Herstellung einer Verbindung der allgemeinen Formel I, die in der 2,3-Stellung eine Doppelbindung besitzt, eine Verbindung der allgemeinen Formel

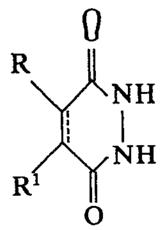

(V)

worin R, $R^1$ und die gestrichelte Linie die oben angegebene Bedeutung besitzen, unter oxydierenden Bedingungen mit einer Verbindung der allgemeinen Formel

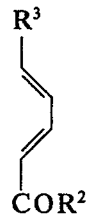

(VIa)

worin $R^2$ und $R^3$ die oben angegebene Bedeutung besitzen, umsetzt, oder

d) zur Herstellung einer Verbindung der allgemeinen Formel I, worin $R^2$ Hydroxy bedeutet und die in der 2,3-Stellung eine Doppelbindung besitzt, eine Verbindung der allgemeinen Formel

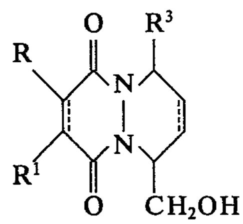

(XVIII)

worin R, $R^1$, $R^3$ und die gestrichelten Linien die oben angegebene Bedeutung besitzen, oxydiert, oder

e) zur Herstellung einer Verbindung der allgemeinen Formel I, worin Y im Rest der Formel (i) $C_2-C_6$-Alkanoylthio, Benzoylthio oder Benzylthio bedeutet, eine Verbindung der allgemeinen Formel

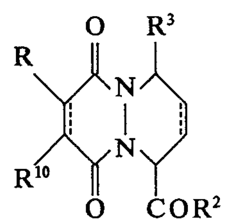

(VII)

worin $R^2$, $R^3$ und die gestrichelten Linien die oben angegebene Bedeutung besitzen und einer der Reste R und $R^{10}$ Wasserstoff oder $C_1-C_6$-Alkyl und der andere einen Rest der Formel

$$-(A)_n-X \qquad \text{(ii)}$$

bedeutet, wobei A und n die oben angegebene Bedeutung besitzen und X Halogen bedeutet, mit einer Verbindung der allgemeinen Formel

$$R^6-SH \qquad \text{(VIII)}$$

worin $R^6$ $C_2-C_6$-Alkanoyl, Benzoyl oder Benzyl bedeutet, umsetzt, oder

f) zur Herstellung einer Verbindung der allgemeinen Formel I, worin Y Mercapto bedeutet, in einer Verbindung der allgemeinen Formel I, worin Y $C_2-C_6$-Alkanoylthio, Benzoylthio oder Benzylthio bedeutet, die $C_2-C_6$-Alkanoyl-, Benzoyl- oder Benzyl-Gruppe abspaltet, oder

g) zur Herstellung einer Verbindung der allgemeinen Formel I, worin R Wasserstoff und $R^1$ einen Rest der Formel (i) bedeutet, wobei A eine Methylengruppe, Y $C_2-C_6$-Alkanoylthio oder Benzoylthio und n die Zahl 1 bedeuten, $R^2$ $C_1-C_6$-Alkoxy und $R^3$ Wasserstoff bedeuten, und die in der 2,3- und der 7,8-Stellung Einfachbindungen besitzt, eine Verbindung der allgemeinen Formel

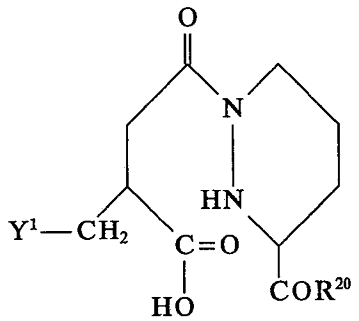

(IX)

worin $R^{20}$ obige Bedeutung besitzt und $Y^1$ $C_2-C_6$-Alkaoylthio oder Benzoylthio bedeutet, cyclisiert, oder

h) zur Herstellung einer Verbindung der allgemeinen Formel I, worin R Wasserstoff und $R^1$ einen Rest der Formel (i) bedeuten, wobei A eine Methylengruppe, Y $C_2-C_6$-Alkanoylthio oder Benzoylthio und n die Zahl 1 bedeuten, und $R^2$ Hydroxy oder $C_1-C_6$-Alkoxy bedeutet und die in der 7,8-Stellung eine Einfachbindung besitzt, eine Verbindung der allgemeinen Formel

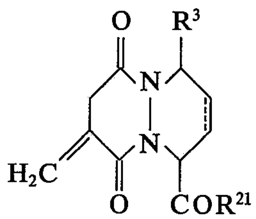

(X)

worin $R^3$ und die gestrichelte Linie die oben angegebene Bedeutung besitzen und $R^{21}$ Hydroxy oder $C_1-C_6$-Alkoxy bedeutet,
mit einer Verbindung der Formel VIII, worin $R^6$ $C_2-C_6$-Alkanoyl oder Benzoyl bedeutet, umsetzt, oder

i) zur Herstellung einer Verbindung der allgemeinen Formel I, worin $R^2$ $C_1-C_6$-Alkoxy oder Amino bedeutet, eine entsprechende Verbindung der allgemeinen Formel I, worin $R^2$ Hydroxy bedeutet, verestert oder amidiert, oder

j) zur Herstellung einer Verbindung der allgemeinen Formel I, worin $R^2$ Hydroxy bedeutet, eine entsprechende Verbindung der allgemeinen Formel I, worin $R^2$ $C_1-C_6$-Alkoxy bedeutet, mit einer Säure oder einer Base behandelt, oder

k) erwünschtenfalls ein erhaltenes Gemisch von Diastereoisomeren in die Racemate auftrennt, und/oder

l) erwünschtenfalls ein erhaltenes Racemat in die optischen Antipoden auftrennt, und/oder

m) erwünschtenfalls eine Carbonsäure der allgemeinen Formel I in ein Salz mit einer pharmazeutisch annehmbaren Base umwandelt.

2. Verfahren gemäß Anspruch 1, dadurch gekennzeichnet, daß man nach den Verfahrensvarianten a), b), c), d), e), f), i) oder j) verfährt und erforderlichenfalls noch die Varianten k), l) oder m) anschließt.

3. Verfahren nach Anspruch 1 oder 2, dadurch gekennzeichnet, daß man Verbindungen der in Anspruch 1 definierten allgemeinen Formel I, worin R Wasserstoff, $R^1$ einen Rest der Formel (i), $R^2$ Hydroxy oder $C_1-C_6$-Alkoxy und $R^3$ Wasserstoff bedeuten und die in 7,8-Stellung eine Einfachbindung besitzen, herstellt.

4. Verfahren nach Anspruch 3, dadurch gekennzeichnet, daß man Verbindungen der in Anspruch 1 definierten allgemeinen Formel I, worin der Rest der Formel (i) Mercaptomethyl oder $C_2-C_6$-Alkanoylthiomethyl bedeutet, herstellt.

5. Verfahren nach Anspruch 1, dadurch gekennzeichnet, daß man (rac.)-Methyl-8-(acetylthio)methyl-octahydro-6,9-dioxopyridazo[1,2-a]pyridazin-1-carboxylat herstellt.

6. Verfahren nach Anspruch 1, dadurch gekennzeichnet, daß man (rac.)-8-(Mercaptomethyl)-octahydro-6,9-dioxopyridazo[1,2-a]pyridazin-1-carbonsäure herstellt.

**Claims for the Contracting States: BE, CH, DE, FR, GB, IT, LI, LU, NL, SE**

1. Pyridazopyridazine derivatives of the general formula

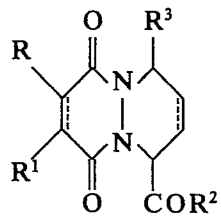

(I)

wherein one of the residues R and $R^1$ signifies hydrogen or $C_1-C_6$-alkyl and the other signifies a residue of the formula

$$-(A)_n-Y \qquad (i)$$

whereby A signifies a methylene, ethylene or propylene group optionally substituted by $C_1-C_6$-alkyl, Y signifies mercapto, $C_2-C_6$-alkanoylthio, benzoylthio or benzylthio and n signifies the number 0 or 1, $R^2$ signifies hydroxy, $C_1-C_6$-alkoxy or amino, $R^3$ signifies hydrogen, $C_1-C_6$-alkyl or phenyl optionally substituted by $C_1-C_6$-alkyl, $C_1-C_6$-alkoxy, halogen or trifluoromethyl and the broken lines signify optional bonds,
and salts of carboxylic acids of general formula I with pharmaceutically acceptable bases.

2. Pyridazopyridazine derivatives in accordance with claim 1, wherein R signifies hydrogen, $R^1$ signifies a residue of formula (i), $R^2$ signifies hydroxy or $C_1-C_6$-alkoxy and $R^3$ signifies hydrogen and which have a single bond in the 7,8-position.

3. Pyridazopyridazine derivatives in accordance with claim 2, wherein the residue of formula (i) signifies mercaptomethyl or $C_2-C_6$-alkanoylthiomethyl.

4. (Rac.) methyl 8-(acetylthio)methyl-octahydro-6,9-dioxopyridazo[1,2-a]pyridazine-1-carboxylate.

5. (Rac.) 8-(mercaptomethyl)-octahydro-6,9-dioxopyridazo[1,2-a]pyridazine-1-carboxylic acid.

6. Pyridazopyridazine derivatives in accordance with any one of claims 1—5 and, if desired, their salts with pharmaceutically acceptable bases for use as pharmaceutically active substances.

7. Pyridazopyridazine derivatives in accordance with any one of claims 1—5 and, if desired, their salts with pharmaceutically acceptable bases for use as antihypertensives.

8. Process for the manufacture of pyridazopyridazine derivatives of general formula I defined in claim 1 and their salts with pharmaceutically acceptable bases, which comprises

a) for the manufacture of a compound of general formula I wherein $R^2$ signifies $C_1-C_6$-alkoxy and $R^3$ signifies hydrogen and which has single bonds in the 2,3- and the 7,8-position, cyclizing a compound of the general formula

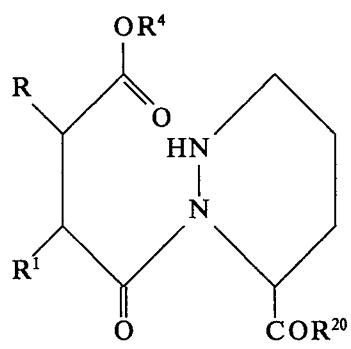

(II)

wherein R and $R^1$ have the significance given in claim 1, $R^4$ signifies hydrogen or $C_1-C_6$-alkyl and $R^{20}$ signifies $C_1-C_6$-alkoxy,

b) for the manufacture of a compound of general formula I wherein $R^2$ signifies hydroxy and $R^3$ signifies hydrogen and which has a single bond in the 2,3-position, reacting the compound of the formula

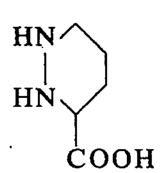

(III)

with an anhydride of the general formula

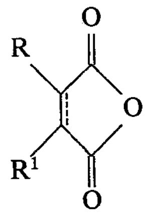

(IV)

wherein R, $R^1$ and the broken line have the significance given in claim 1,

or

c) for the manufacture of a compound of general formula I which has a double bond in the 2,3-position, reacting a compound of the general formula

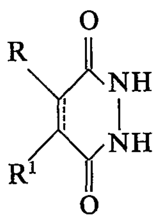

(V)

wherein R, $R^1$ and the broken line have the significance given in claim 1, under oxidizing conditions with a compound of the general formula

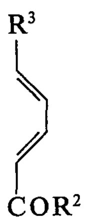

(VIa)

wherein $R^2$ and $R^3$ have the significance given in claim 1,

or

d) for the manufacture of a compound of general formula I wherein $R^2$ signifies hydroxy and which has a double bond in the 2,3-position, oxidizing a compound of the general formula

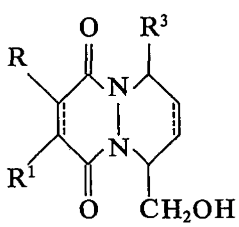

(XVIII)

wherein R, $R^1$, $R^3$ and the broken lines have the significance given in claim 1,

or

e) for the manufacture of a compound of general formula I wherein Y in the residue of formula (i) signifies $C_2-C_6$-alkanoylthio, benzoylthio or benzylthio, reacting a compound of the general formula

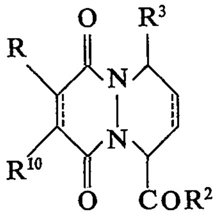

(VII)

wherein $R^2$, $R^3$ and the broken lines have the significance given in claim 1 and one of the residues R and $R^{10}$ signifies hydrogen or $C_1-C_6$-alkyl and the other signifies a residue of the formula

$-(A)_n-X$ (ii)

whereby A and n have the significance given in claim 1 and X signifies halogen, with a compound of the general formula

$R^6-SH$ (VIII)

wherein $R^6$ signifies $C_2-C_6$-alkanoyl, benzoyl or benzyl, or

f) for the manufacture of a compound of general formula I wherein Y signifies mercapto, cleaving off the $C_2-C_6$-alkanoyl, benzoyl or benzyl group in a compound of general formula I wherein Y signifies $C_2-C_6$-alkanoylthio, benzoylthio or benzylthio, or

g) for the manufacture of a compound of general formula I wherein R signifies hydrogen and $R^1$ signifies a residue of formula (i), whereby A signifies a methylene group, Y signifies $C_2-C_6$-alkanoylthio or benzoylthio and n signifies the number 1, $R^2$ signifies $C_1-C_6$-alkoxy and $R^3$ signifies hydrogen, and which has single bonds in the 2,3- and the 7,8-position, cyclizing a compound of the general formula

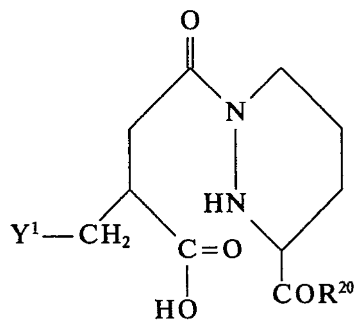

(IX)

wherein $R^{20}$ has the above significance and $Y^1$ signifies $C_2-C_6$-alkanoylthio or benzoylthio, or

h) for the manufacture of a compound of general formula I wherein R signifies hydrogen and $R^1$ signifies a residue of formula (i), whereby A signifies a methylene group, Y signifies $C_2-C_6$-alkanoylthio or benzoylthio and n signifies the number 1, and $R^2$ signifies hydroxy or $C_1-C_6$-alkoxy and which has a single bond in the 7,8-position, reacting a compound of the general formula

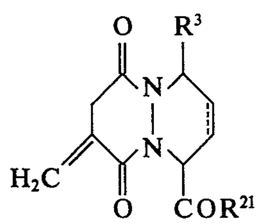

(X)

wherein $R^3$ and the broken line have the significance given in claim 1 and $R^{21}$ signifies hydroxy or $C_1-C_6$-alkoxy,
with a compound of formula VIII wherein $R^6$ signifies $C_2-C_6$-alkanoyl or benzoyl, or

i) for the manufacture of a compound of general formula I wherein $R^2$ signifies $C_1-C_6$-alkoxy or amino, esterifying or amidating a corresponding compound of general formula I wherein $R^2$ signifies hydroxy, or

j) for the manufacture of a compound of general formula I wherein $R^2$ signifies hydroxy, treating a corresponding compound of general formula I wherein $R^2$ signifies $C_1-C_6$-alkoxy with an acid or a base, or

k) if desired, separating a mixture of diastereoisomers obtained into the racemates, and/or

l) if desired, resolving a racemate obtained into the optical antipodes, and/or

m) if desired, converting a carboxylic acid of general formula I into a salt with a pharmaceutically acceptable base.

9. Process in accordance with claim 8, characterized in that one proceeds according to process variants a), b), c), d), e), f), i) or j) and, if required, appends variants k), l) or m).

10. Medicament, containing a pyridazopyridazine derivative in accordance with any one of claims 1 – 5 or, if desired, a salt thereof with a pharmaceutically acceptable base.

11. Antihypertensive, containing a pyridazopyridazine derivative in accordance with any one of claims 1 – 5 or, if desired, a salt thereof with a pharmaceutically acceptable base.

**Claims for the Contracting State: AT**

1. Process for the manufacture of pyridazopyridazine derivatives of the general formula

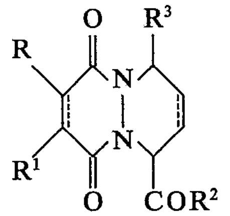

(I)

wherein one of the residues R and $R^1$ signifies hydrogen or $C_1-C_6$-alkyl and the other signifies a residue of the formula

$$-(A)_n-Y \qquad (i)$$

whereby A signifies a methylene, ethylene or propylene group optionally substituted by $C_1-C_6$-alkyl, Y signifies mercapto, $C_2-C_6$-alkanoylthio, benzoylthio or benzylthio and n signifies the number 0 or 1, $R^2$ signifies hydroxy, $C_1-C_6$-alkoxy or amino, $R^3$ signifies hydrogen, $C_1-C_6$-alkyl or phenyl optionally substituted by $C_1-C_6$-alkyl, $C_1-C_6$-alkoxy, halogen or trifluoromethyl and the broken lines signify optional bonds,
and salts of carboxylic acids of general formula I with pharmaceutically acceptable bases, which comprises

a) for the manufacture of a compound of general formula I wherein $R^2$ signifies $C_1-C_6$-alkoxy and $R^3$ signifies hydrogen and which has single bonds in the 2,3- and the 7,8-position, cyclizing a compound of the general formula

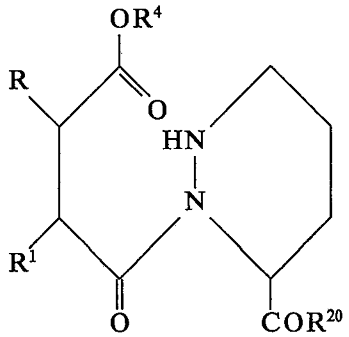

(II)

wherein R and $R^1$ have the significance given above, $R^4$ signifies hydrogen or $C_1-C_6$-alkyl and $R^{20}$ signifies $C_1-C_6$-alkoxy,
or

b) for the manufacture of a compound of general formula I wherein $R^2$ signifies hydroxy and $R^3$ signifies hydrogen and which has a single bond in the 2,3-position, reacting the compound of the formula

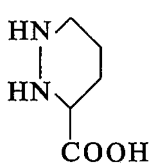

(III)

with an anhydride of the general formula

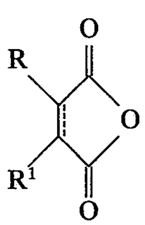

(IV)

wherein R, $R^1$ and the broken line have the significance given above,
or

c) for the manufacture of a compound of general formula I which has a double bond in the 2,3-position, reacting a compound of the general formula

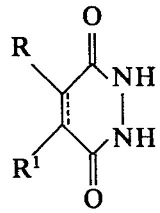

(V)

wherein R, $R^1$ and the broken line have the significance given above, under oxidizing conditions with a compound of the general formula

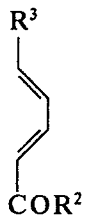

(VIa)

wherein $R^2$ and $R^3$ habe the significance given above,
or

d) for the manufacture of a compound of general formula I wherein $R^2$ signifies hydroxy and which has a double bond in the 2,3-position, oxidizing a compound of the general formula

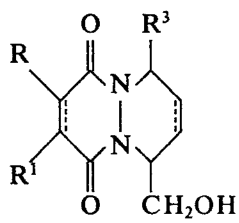

(XVIII)

wherein R, $R^1$, $R^3$ and the broken lines have the significance given above,
or

e) for the manufacture of a compound of general formula I wherein Y in the residue of formula (i) signifies $C_2-C_6$-alkanoylthio, benzoylthio or benzylthio, reacting a compound of the general formula

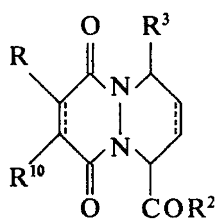

(VII)

wherein $R^2$, $R^3$ and the broken lines have the significance given above and one of the residues R and $R^{10}$ signifies hydrogen or $C_1-C_6$-alkyl and the other signifies a residue of the formula

$$-(A)_n-X \quad \text{(ii)}$$

whereby A and n have the significance given above and X signifies halogen, with a compound of the general formula

$$R^6-SH \quad \text{(VIII)}$$

wherein $R^6$ signifies $C_2-C_6$-alkanoyl, benzoyl or benzyl,
or

f) for the manufacture of a compound of general formula I wherein Y signifies mercapto, cleaving off the $C_2-C_6$-alkanoyl, benzoyl or benzyl group in a compound of general formula I wherein Y signifies $C_2-C_6$-alkanoylthio, benzoylthio or benzylthio, or

g) for the manufacture of a compound of general formula I wherein R signifies hydrogen and $R^1$ signifies a residue of formula (i), whereby A signifies a methylene group, Y signifies

$C_2-C_6$-alkanoylthio or benzylthio and n signifies the number 1, $R^2$ signifies $C_1-C_6$-alkoxy and $R^3$ signifies hydrogen, and which has single bonds in the 2,3- and the 7,8-position, cyclizing a compound of the general formula

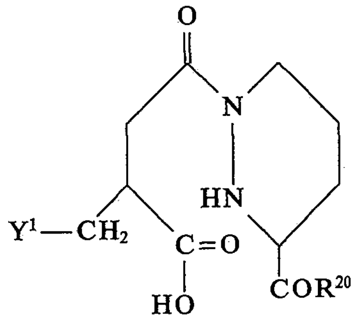

(IX)

wherein $R^{20}$ has the above significance and $Y^1$ signifies $C_2-C_6$-alkanoylthio or benzoylthio, or

h) for the manufacture of a compound of general formula I wherein R signifies hydrogen and $R^1$ signifies a residue of formula (i), whereby A signifies a methylene group, Y signifies $C_2-C_6$-alkanoylthio or benzoylthio and n signifies the number 1, and $R^2$ signifies hydroxy or $C_1-C_6$-alkoxy and which has a single bond in the 7,8-position, reacting a compound of the general formula

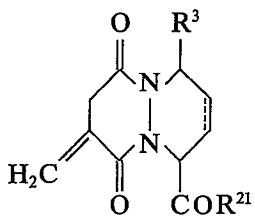

(X)

wherein $R^3$ and the broken line have the significance given above and $R^{21}$ signifies hydroxy or $C_1-C_6$-alkoxy,
with a compound of formula VIII wherein $R^6$ signifies $C_2-C_6$-alkanoyl or benzoyl, or

i) for the manufacture of a compound of general formula I wherein $R^2$ signifies $C_1-C_6$-alkoxy or amino, esterifying or amidating a corresponding compound of general formula I wherein $R^2$ signifies hydroxy, or

j) for the manufacture of a compound of general formula I wherein $R^2$ signifies hydroxy, treating a corresponding compound of general formula I wherein $R^2$ signifies $C_1-C_6$-alkoxy with an acid or a base, or

k) if desired, separating a mixture of diastereoisomers obtained into the racemates, and/or

l) if desired, resolving a racemate obtained into the optical antipodes, and/or

m) if desired, converting a carboxylic acid of general formula I into a salt with a pharmaceutically acceptable base.

2. Process in accordance with claim 1, characterized in that one proceeds according to process variants a), b), c), d), e), f), i) or j) and, if required, appends variants k), l) or m).

3. Process in accordance with claim 1 or 2, characterized in that there are manufactured compounds of general formula I defined in claim 1 wherein R signifies hydrogen, $R^1$ signifies a residue of formula (i), $R^2$ signifies hydroxy or $C_1-C_6$-alkoxy and $R^3$ signifies hydrogen and which have a single bond in the 7,8-position.

4. Process in accordance with claim 3, characterized in that there are manufactured compounds of general formula I defined in claim 1 wherein the residue of formula (i) signifies mercaptomethyl or $C_2-C_6$-alkanoylthiomethyl.

5. Process in accordance with claim 1, characterized in that (rac.) methyl 8-(acetylthio)methyl-octahydro-6,9-dioxopyridazo[1,2-a]pyridazine-1-carboxylate is manufactured.

6. Process in accordance with claim 1, characterized in that (rac.) 8-(mercaptomethyl)-octahydro-6,9-dioxopyridazo[1,2-a]pyridazine-1-carboxylic acid is manufactured.

**Revendications pour les Etats contractants: BE, CH, DE, FR, GB, IT, LI, LU, NL, SE**

1. Dérivés de pyridazopyridazine de formule générale

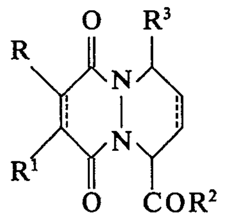

(I)

dans laquelle l'un des symboles R et $R^1$ représente un atome d'hydrogène ou un groupe alcoyle en $C_1-C_6$ et l'autre symbole représente un groupe de formule

$$-(A)_n-Y \qquad (i)$$

dans laquelle A représente un groupe méthylène, éthylène ou propylène qui peut être substitué par un groupe alcoyle en $C_1-C_6$, Y représente un groupe mercapto, (alcanoyl en $C_2-C_6$)thio, benzoylthio ou benzylthio et n représente zéro ou 1, $R^2$ représente un groupe hydroxy, alcoxy en $C_1-C_6$ ou amino, $R^3$ représente un atome d'hydrogène ou un groupe alcoyle en $C_1-C_6$ ou phényle qui peut être substitué par un groupe alcoyle en $C_1-C_6$, alcoxy en $C_1-C_6$, par un halogène ou par un groupe trifluorométhyle et les lignes formées de tirets indiquent des liaisons éventuelles,
et les sels des acides carboxyliques de formule générale I avec des bases pharmaceutiquement acceptables.

2. Dérivés de pyridazopyridazine selon la revendication 1, dans lesquels R représente un atome d'hydrogène, $R^1$ représente un groupe de formule (i), $R^2$ représente un groupe hydroxy ou alcoxy en $C_1-C_6$, $R^3$ représente un atome d'hydrogène et une liaison simple est présente dans la position 7, 8.

3. Dérivés de pyridazopyridazine selon la revendication 2, dans lesquels le groupe de formule (i) est le groupe mercaptométhyle ou un groupe (alcanoyl en $C_2-C_6$)thiométhyle.

4. 8-(Acétylthio)méthyl-octahydro-6,9-dioxopyridazo[1,2-a]pyridazine-1-carboxylate de méthyle racémique.

5. Acide 8-(mercaptométhyl)-octahydro-6,9-dioxopyridazo[1,2-a]pyridazine-1-carboxylique racémique.

6. Dérivés de pyridazopyridazine selon l'une des revendications 1 à 5 et éventuellement leurs sels avec des bases pharmaceutiquement acceptables pour utilisation comme substances actives pharmaceutiques.

7. Dérivés de pyridazopyridazine selon l'une des revendications 1 à 5 et éventuellement leurs sels avec des bases pharmaceutiquement acceptables pour utilisation comme antihypertensifs.

8. Un procédé pour la préparation des dérivés de pyridazopyridazine de formule générale I définie dans la revendication 1 et de leurs sels avec des bases pharmaceutiquement acceptables, caractérisé en ce que:

(a) pour la préparation d'un composé de formule générale I dans laquelle $R^2$ représente un groupe alcoxy en $C_1-C_6$, $R^3$ représente un atome d'hydrogène et des liaisons simples sont présentes dans les positions 2,3 et 7,8, on cyclise un composé de formule générale

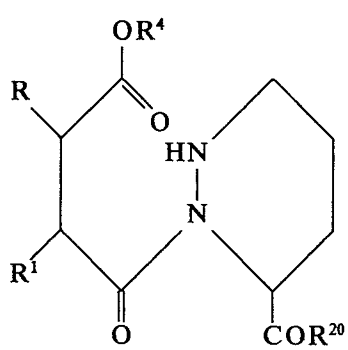

(II)

dans laquelle R et $R^1$ ont la signification indiquée dans la revendication 1, $R^4$ représente un atome d'hydrogène ou un groupe alcoyle en $C_1-C_6$ et $R^{20}$ représente un groupe alcoxy en $C_1-C_6$, ou

(b) pour la préparation d'un composé de formule générale I dans laquelle $R^2$ représente un groupe hydroxy, $R^3$ représente un atome d'hydrogène et une liaison simple est présente dans la position 2,3, on fait réagir le composé de formule

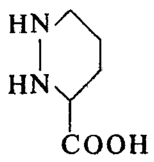

(III)

avec un anhydride de formule générale

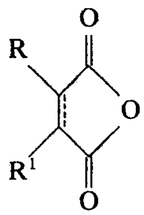

(IV)

dans laquelle R, $R^1$ et la ligne formée de tirets ont la signification indiquée dans la revendication 1, ou

(c) pour la préparation d'un composé de formule générale I dans laquelle une double liaison est présente dans la position 2,3, on fait réagir un composé de formule générale

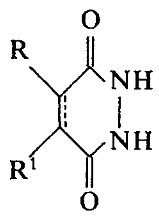

(V)

dans laquelle R, $R^1$ et la ligne sormée de tirets ont la signification indiquée dans la revendication 1, dans des conditions oxydantes, avec un composé de formule générale

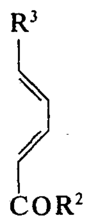

(VI a)

dans laquelle $R^2$ et $R^3$ ont la signification indiquée dans la revendication 1, ou

(d) pour la préparation d'un composé de formule générale I dans laquelle $R^2$ représente un groupe hydroxy et une double liaison est présente dans la position 2,3, on oxyde un composé de formule générale

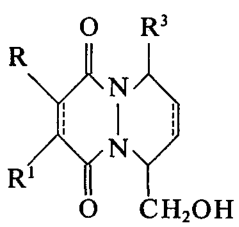

(XVIII)

dans laquelle R, $R^1$, $R^3$ et les lignes formées de tirets ont la signification indiquée dans la revendication 1, ou

(e) pour la préparation d'un composé de formule générale I dans laquelle Y dans le groupe de formule (i) représente un groupe (alcanoyl en $C_2-C_6$)thio, benzoylthio ou benzylthio, on fait réagir un composé de formule générale

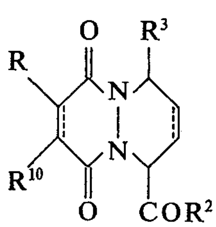

(VII)

dans laquelle $R^2$, $R^3$ et les lignes formées de tirets ont la signification indiquée dans la revendication 1 et un des symboles R et $R^{10}$ représente un atome d'hydrogène ou un groupe alcoyle en $C_1-C_6$ et l'autre symbole représente un groupe de formule

$$-(A)_n-X \qquad \text{(ii)}$$

dans laquelle A et n ont la signification indiquée dans la revendication 1 et X représente un atome d'halogène,
avec un composé de formule générale

$$R^6-SH \qquad \text{(VIII)}$$

dans laquelle $R^6$ représente un groupe alcanoyle en $C_2-C_6$, benzoyle ou benzyle, ou

(f) pour la préparation d'un composé de formule générale I dans laquelle Y représente un groupe mercapto, on élimine le groupe alcanoyle en $C_2-C_6$, benzoyle ou benzyle d'un composé de formule générale I dans laquelle Y représente un groupe (alcanoyl en $C_2-C_6$)thio, benzoylthio ou benzylthio, ou

(g) pour la préparation d'un composé de formule générale I dans laquelle R représente un atome d'hydrogène, $R^1$ représente un groupe de formule (i) dans laquelle A représente un groupe méthylène, Y représente un groupe (alcanoyl en $C_2-C_6$)thio ou benzoylthio et n représente 1, $R^2$ représente un groupe alcoxy en $C_1-C_6$, $R^3$ représente un atome d'hydrogène et des liaisons simples sont présentes dans les positions 2,3 et 7,8, on cyclise un composé de formule générale

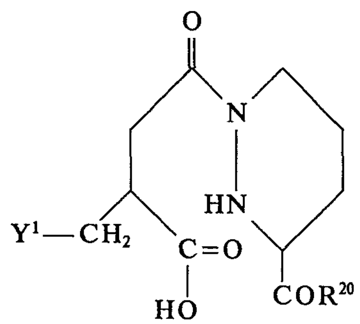

(IX)

dans laquelle $R^{20}$ a la signification indiquée plus haut et $Y^1$ représente un groupe (alcanoyl en $C_2-C_6$)thio ou benzoylthio, ou

(h) pour la préparation d'un composé de formule générale I dans laquelle R représente un atome d'hydrogène, $R^1$ représente un groupe de formule (i) dans laquelle A représente un groupe méthylène, Y représente un groupe (alcanoyl en $C_2-C_6$)thio ou benzoylthio et n représente 1, $R^2$ représente un groupe hydroxy ou alcoxy en $C_1-C_6$ et une liaison simple est présente dans la position 7,8, on fait réagir un composé de formule générale

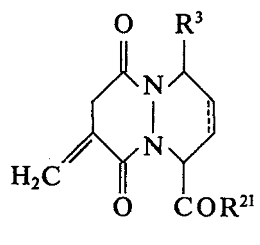

(X)

dans laquelle $R^3$ et la ligne formée de tirets ont la signification indiquée dans la revendication 1 et $R^{21}$ représente un groupe hydroxy ou alcoxy en $C_1-C_6$, avec un composé de formule VIII dans laquelle $R^6$ représente un groupe alcanoyle en $C_2-C_6$ ou benzoyle, ou

(i) pour la préparation d'un composé de formule générale I dans laquelle $R^2$ représente un groupe alcoxy en $C_1-C_6$ ou amino, on estérifie ou on amidifie un composé correspondant de formule générale I dans laquelle $R^2$ représente un groupe hydroxy, ou

(j) pour la préparation d'un composé de formule générale I dans laquelle $R^2$ représente un groupe hydroxy, on traite un composé correspondant de formule générale I dans laquelle $R^2$ représente un groupe alcoxy en $C_1-C_6$ par un acide ou une base, ou

(k) si on le désire, on sépare un mélange de diastéréoisomères obtenu en ses racémates, et/ou

(l) si on le désire, on dédouble un racémate obtenu en ses antipodes optiques, et/ou

(m) si on le désire, on transforme un acide carboxylique de formule générale I en un sel avec une base pharmaceutiquement acceptable.

9. Un procédé selon la revendication 8, caractérisé en ce qu'on utilise l'un des modes de mise en

eouvre (a), (b), (c), (d), (e), (f), (i) ou (j), et l'on procède ensuite si nécessaire selon les variantes (k), (l) ou (m).

10. Médicament contenant un dérivé de pyridazopyridazine selon l'une des revendications 1 à 5 ou é éventuellement l'un de ses sels avec une base pharmaceutiquement acceptable.

11. Antihypertensif contenant un dérivé de pyridazopyridazine selon l'une des revendications 1 à 5 ou éventuellement l'un de ses sels avec une base pharmaceutiquement acceptable.

**Revendications pour l'Etat contractant: AT**

1. Un procédé pour la préparation de dérivés de pyridazopyridazine de formule générale

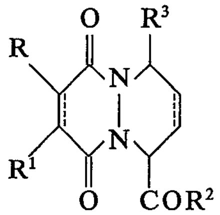

(I)

dans laquelle l'un des symboles R et $R^1$ représente un atome d'hydrogène ou un groupe alcoyle en $C_1-C_6$ et l'autre symbole représente un groupe de formule

$$-(A)_n-Y \quad \text{(i)}$$

dans laquelle A représente un groupe méthylène, éthylène ou propylène qui peut être substitué par un groupe alcoyle en $C_1-C_6$, Y représente un groupe mercapto, (alcanoyl en $C_2-C_6$)thio, benzoylthio ou benzylthio et n représente zéro ou 1, $R^2$ représente un groupe hydroxy, alcoxy en $C_1-C_6$ ou amino, $R^3$ représente un atome d'hydrogène ou un groupe alcoyle en $C_1-C_6$ ou phényle qui peut être substitué par un groupe alcoyle en $C_1-C_6$, alcoxy en $C_1-C_6$, par un halogène ou par un groupe trifluorométhyle et les lignes formées de tirets indiquent des liaisons éventuelles,
ainsi que des sels des acides carboxyliques de formule générale I avec des bases pharmaceutiquement acceptables, caractérisé en ce que:

(a) pour la préparation d'un composé de formule générale I dans laquelle $R^2$ représente un groupe alcoxy en $C_1-C_6$, $R^3$ représente un atome d'hydrogène et des liaisons simples sont présentes dans les positions 2,3 et 7,8, on cyclise un composé de formule générale

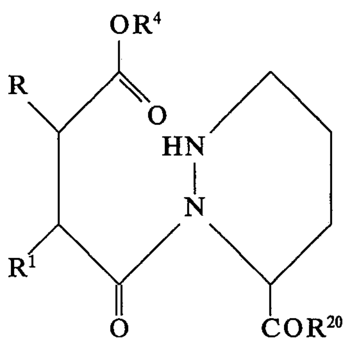

(II)

dans laquelle R et $R^1$ ont la signification indiquée plus haut, $R^4$ représente un atome d'hydrogène ou un groupe alcoyle en $C_1-C_6$ et $R^{20}$ représente un groupe alcoxy en $C_1-C_6$, ou

(b) pour la préparation d'un composé de formule générale I dans laquelle $R^2$ représente un groupe hydroxy, $R^3$ représente un atome d'hydrogène et une liaison simple est présente dans la position 2,3, on fait réagir le composé de formule

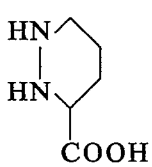

(III)

avec un anhydride de formule générale

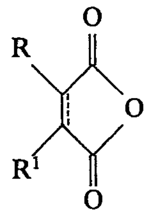

(IV)

dans laquelle R, $R^1$ et la ligne formée de tirets ont la signification indiquée plus haut, ou

(c) pour la préparation d'un composé de formule générale I dans laquelle une double liaison est présente dans la position 2,3, on fait réagir un composé de formule générale

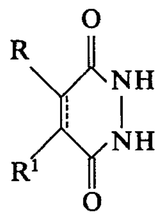

(V)

dans laquelle R, $R^1$ et la ligne formée de tirets ont la signification indiquée plus haut, dans des conditions oxydantes, avec un composé de formule générale

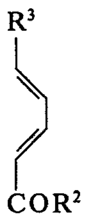

(VI a)

dans laquelle $R^2$ et $R^3$ ont la signification indiquée plus haut, ou

(d) pour la préparation d'un composé de formule générale I dans laquelle $R^2$ représente un groupe hydroxy et une double liaison est présente dans la position 2,3, on oxyde un composé de formule générale

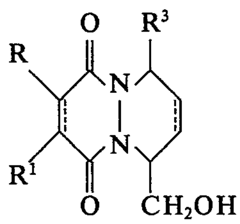

(XVIII)

dans laquelle R, $R^1$, $R^3$ et les lignes formées de tirets ont la signification indiquée plus haut, ou

(e) pour la préparation d'un composé de formule générale I dans laquelle Y dans le groupe de formule (i) représente un groupe (alcanoyl en $C_2-C_6$)thio, benzoylthio ou benzylthio, on fait réagir un composé de formule générale

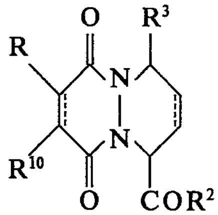

(VII)

dans laquelle $R^2$, $R^3$ et les lignes formées de tirets ont la signification indiquée plus haut et un des symboles R et $R^{10}$ représente un atome d'hydrogène ou un groupe alcoyle en $C_1-C_6$ et l'autre symbole représente un groupe de formule

$$-(A)_n-X \qquad \text{(ii)}$$

dans laquelle A et n ont la signification indiquée plus haut et X représente un atome d'halogène, avec un composé de formule générale

$R^6-SH$ (VIII)

dans laquelle $R^6$ représente un groupe alcanoyle en $C_2-C_6$, benzoyle ou benzyle, ou

(f) pour la préparation d'un composé de formule générale I dans laquelle Y représente un groupe mercapto, on élimine le groupe alcanoyle en $C_2-C_6$, benzoyle ou benzyle d'un composé de formule générale I dans laquelle Y représente un groupe (alcanoyl en $C_2-C_6$)thio, benzoylthio ou benzylthio, ou

(g) pour la préparation d'un composé de formule générale I dans laquelle R représente un atome d'hydrogène, $R^1$ représente un groupe de formule (i) dans laquelle A représente un groupe méthylène, Y représente un groupe (alcanoyl en $C_2-C_6$)thio ou benzoylthio et n représente 1, $R^2$ représente un groupe alcoxy en $C_1-C_6$, $R^3$ représente un atome d'hydrogène et des liaisons simples sont présentes dans les positions 2,3 et 7,8, on cyclise un composé de formule générale

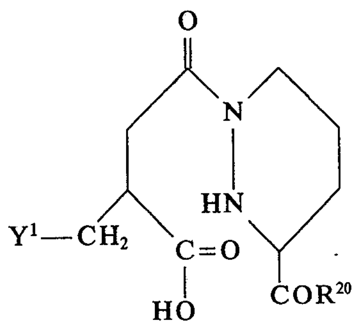

(IX)

dans laquelle $R^{20}$ a la signification indiquée plus haut et $Y^1$ représente un groupe (alcanoyl en $C_2-C_6$)thio ou benzoylthio, ou

(h) pour la préparation d'un composé de formule générale I dans laquelle R représente un atome d'hydrogène, $R^1$ représente un groupe de formule (i) dans laquelle A représente un groupe méthylène, Y représente un groupe (alcanoyl en $C_2-C_6$)thio ou benzoylthio et n représente 1, $R^2$ représente un groupe hydroxy ou alcoxy en $C_1-C_6$ et une liaison simple est présente dans la position 7,8, on fait réagir un composé de formule générale

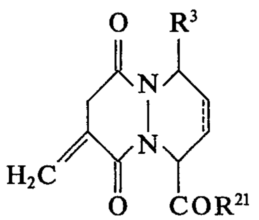

(X)

dans laquelle $R^3$ et la ligne formée de tirets ont la signification indiquée plus haut et $R^{21}$ représente un groupe hydroxy ou alcoxy en $C_1-C_6$,
avec un composé de formule VIII dans laquelle $R^6$ représente un groupe alcanoyle en $C_2-C_6$ ou benzoyle, ou

(i) pour la préparation d'un composé de formule générale I dans laquelle $R^2$ représente un groupe alcoxy en $C_1-C_6$ ou amino, on estérifie ou on amidifie un composé correspondant de formule générale I dans laquelle $R^2$ représente un groupe hydroxy, ou

(j) pour la préparation d'un composé de formule générale I dans laquelle $R^2$ représente un groupe hydroxy, on traite un composé correspondant de formule générale I dans laquelle $R^2$ représente un groupe alcoxy en $C_1-C_6$ par un acide ou une base,

(k) si on le désire, on sépare un mélange de diastéréoisomères obtenu en ses racémates, et/ou

(l) si on le désire, on dédouble un racémate obtenu en ses antipodes optiques, et/ou

(m) si on le désire, on transforme un acide carboxylique de formule générale I en un sel avec une base pharmaceutiquement acceptable.

2. Un procédé selon la revendication 1, caractérisé en ce qu'on utilise l'un des modes de mise en œuvre (a), (b), (c), (d), (e), (f), (i) ou (j), et l'on procède ensuite si nécessaire selon les variantes (k), (l) ou (m).

3. Un procédé selon l'une des revendications 1 ou 2, caractérisé en ce qu'on prépare des composés de formule générale I définie dans la revendication 1, dans laquelle R représente un atome d'hydrogène, $R^1$ représente un groupe de formule (i), $R^2$ représente un groupe hydroxy ou alcoxy en $C_1-C_6$, $R^3$ représente un atome d'hydrogène et une liaison simple est présente dans la position 7,8.

4. Un procédé selon la revendication 3, caractérisé en ce qu'on prépare des composés de formule générale I définie dans la revendication 1, dans laquelle le groupe de formule (i) est le groupe mercaptométhyle ou un groupe (alcanoyl en $C_2-C_6$)thiométhyle.

5. Un procédé selon la revendication 1, caractérisé en ce qu'on prépare le 8-(acétylthio)méthyloctahydro-6,9-dioxopyridazo[1,2-a]pyridazine-1-carboxylate de méthyle racémique.

6. Un procédé selon la revendication 1, caractérisé en ce qu'on prépare l'acide 8-(mercaptométhyl)-octahydro-6,9-dioxopyridazo[1,2-a]-pyridazine-1-carboxylique racémique.